(19) **Europäisches Patentamt European Patent Office Office européen des brevets**

(11) **EP 4 296 347 B1**

(12) # EUROPEAN PATENT SPECIFICATION

(45) Date of publication and mention of the grant of the patent:
**29.04.2026 Bulletin 2026/18**

(21) Application number: **21926737.4**

(22) Date of filing: **30.11.2021**

(51) International Patent Classification (IPC):
*C12M 1/34* (2006.01)     *G01N 33/50* (2006.01)
*G01N 33/483* (2006.01)

(52) Cooperative Patent Classification (CPC):
**G16H 40/63; C12M 1/34; C12N 5/06;**
**G01N 33/4836;** G01N 33/5061; G01N 2500/10

(86) International application number:
**PCT/JP2021/043955**

(87) International publication number:
**WO 2022/176310 (25.08.2022 Gazette 2022/34)**

(54) **INFORMATION PROCESSING DEVICE, INFORMATION PROCESSING METHOD AND PROGRAM**

INFORMATIONSVERARBEITUNGSVORRICHTUNG, INFORMATIONSVERARBEITUNGSVERFAHREN UND -PROGRAMM

DISPOSITIF DE TRAITEMENT D'INFORMATIONS, PROCÉDÉ DE TRAITEMENT D'INFORMATIONS ET PROGRAMME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.02.2021 JP 2021023760**

(43) Date of publication of application:
**27.12.2023 Bulletin 2023/52**

(73) Proprietor: **FUJIFILM Corporation**
**Tokyo 106-8620 (JP)**

(72) Inventors:
• **INABA, Tatsuya**
 **Ashigarakami-gun, Kanagawa 258-8577 (JP)**
• **HASEGAWA, Masataka**
 **Ashigarakami-gun, Kanagawa 258-8577 (JP)**
• **MIYOSHI, Hayato**
 **Ashigarakami-gun, Kanagawa 258-8577 (JP)**

• **MORIMURA, Kaoru**
 **Ashigarakami-gun, Kanagawa 258-8577 (JP)**

(74) Representative: **Hoffmann Eitle**
**Patent- und Rechtsanwälte PartmbB**
**Arabellastraße 30**
**81925 München (DE)**

(56) References cited:
**WO-A1-2007/058001**     **WO-A1-2019/196076**
**WO-A1-2020/226879**     **JP-A- H05 324 876**
**US-A1- 2018 372 724**     **US-A1- 2020 178 825**

• **CANTWELL CHRIS D ET AL: "Rethinking multiscale cardiac electrophysiology with machine learning and predictive modelling",** COMPUTERS IN BIOLOGY AND MEDICINE, vol. 104, 18 October 2018 (2018-10-18), pages 339 - 351, XP085565128, ISSN: 0010-4825, DOI: 10.1016/J.COMPBIOMED.2018.10.015

**Description**

BACKGROUND OF THE INVENTION

1. Field of the Invention

The technology of the present disclosure relates to an information processing apparatus, an information processing method, a program, and a drug evaluation method.

2. Description of the Related Art

In order to improve an efficiency of new drug development, for example, a method of performing a toxicity evaluation using cells such as myocardial cells produced from induced pluripotent stem (iPS) cells has been developed (see, for example, WO2019/131806A). The toxicity evaluation is performed by evaluating responsiveness of cells to a drug.

As a culture vessel for the myocardial cells, for example, a well plate in which a plurality of wells are formed is used. A microelectrode array (MEA) in which a plurality of microelectrodes are disposed is provided on a bottom surface of each well. Such a well plate is called an MEA plate. A waveform (for example, a myocardial waveform indicating pulsation of the myocardial cells) indicating an electrophysiological change of cells cultured in the wells is output from each microelectrode of the microelectrode array. The toxicity evaluation is performed by measuring a change in waveform with respect to a drug.

Variations occur in the waveform output from each of the plurality of microelectrodes provided in the wells. Therefore, for each well, one microelectrode from which the most ideal waveform is output is selected as a target electrode to be a target for the toxicity evaluation. Such selection of the target electrode is also called selection of the golden channel (see, for example, Reference 1). For example, in a case of the myocardial cells, a waveform closest to the healthiest state is selected among the myocardial waveforms output from the microelectrodes based on a known myocardial waveform representing a healthy state (for example, a state without a disease such as arrhythmia) obtained in the past measurement.

Reference 1: Seeding and culturing of iCell (registered trademark) myocardial cells 2.0 on an MED plate, [Searched on January 27, 2021], Internet <https://alphamedsci.com/download/protocols/dissociated5_190625%20(J).pdf>

WO 2020/226879 A1 discloses a computing device which uses both a classification through feature delineation and a classification through machine learning to detect cardiac arrhythmia in the cardiac electrogram of a patient. If an episode of arrhythmia has occurred, the computing device outputs a report containing the cardiac features related to the episode. WO 2019/196076 A1 discloses systems and methods for determining blood pressure of a subject. Upon receiving a request from a terminal, the method extracts target features from heart activity related data, determines a preliminary blood pressure using a prediction model and uses an optimization model on the preliminary blood pressure to generate a predicted blood pressure.

US 2018/0372724 A1 discloses a platform configured to predict type or family of an unknown drug candidate compound. The platform includes one or more processing units configured to process cellular response data to the drug compound and compare that response to a library of response data, and, based on the similarity of the response, predict the type or family of the unknown compound.

"Rethinking multiscale cardiac electrophysiology with machine learning and predictive modelling" Cantwell et al., 2019, is a review article disclosing the latest approaches to analyzing cardiac electrophysiology data using machine learning and predictive modelling.

SUMMARY OF THE INVENTION

Since there is no clear definition for the ideal waveform that serves as a reference for selecting the target electrode, and only a rough shape is determined, it is difficult to mechanically select the target electrode based on the waveform output from each of the microelectrodes. Therefore, a current state of the art is that the target electrode is selected by a sensory evaluation in which the waveform output from each of the microelectrodes is compared with a known ideal waveform by a human.

However, in the selection of the target electrode based on such a sensory evaluation, there are problems in that the evaluation takes a long time and the evaluation varies depending on an experience of an evaluator. Reference 1 described above discloses that the target electrode is selected based on a peak amplitude of the waveform output from each of the microelectrodes, but it is difficult to select a waveform close to an ideal waveform with high accuracy by such a simple mechanical method.

Therefore, it is desired to develop a method that enables selection of a waveform close to an ideal waveform in a short time while maintaining the selection accuracy of the waveform by the sensory evaluation so far. Such a problem regarding selection of a waveform close to an ideal waveform is not limited to the field related to the toxicity evaluation, and exists in various fields.

An object of the technology of the present disclosure is to provide an information processing apparatus, an information processing method, and a program that enable selection of a waveform close to an ideal waveform with high accuracy in a short time.

This object is accomplished by the subject-matter of the independent claims. The dependent claims concern particular embodiments.

According to the technology of the present disclosure, it is possible to provide an information processing apparatus, an information processing method, and a program that enable selection of a waveform close to an ideal waveform with high accuracy in a short time.

BRIEF DESCRIPTION OF THE DRAWINGS

Fig. 1 is a diagram schematically showing an electrical function evaluation system.
Fig. 2 is a perspective view showing an example of an MEA plate.
Fig. 3 is a diagram showing an example of a well.
Fig. 4 is a diagram showing an example of a microelectrode array.
Fig. 5 is a block diagram showing an example of a hardware configuration of the electrical function evaluation system.
Fig. 6 is a block diagram showing an example of a functional configuration of an information processing apparatus.
Fig. 7 is a diagram showing an example of a myocardial waveform.
Fig. 8 is a diagram showing an example of unknown waveform data output from the microelectrode array.
Fig. 9 is a flowchart showing an example of a determination process.
Fig. 10 is a diagram showing an example of a set including a plurality of unknown waveform data and a plurality of teacher waveform data.
Fig. 11 is a diagram showing an example of clustering.
Fig. 12 is a diagram illustrating a method of clustering.
Fig. 13 is a diagram illustrating a method of calculating a score.
Fig. 14 is a diagram showing an example of ranking of unknown waveform data ranked based on a score.
Fig. 15 is a diagram showing an example of final ranking after re-clustering.
Fig. 16 is a diagram showing an example in which superiority or inferiority of a plurality of unknown waveform data is displayed in a comparable manner.
Fig. 17 is a diagram showing a set including a plurality of teacher waveform data and a plurality of unknown waveform data prepared in Examples.
Fig. 18 is a diagram showing results of first clustering performed in Examples.
Fig. 19 is a diagram showing results of second clustering performed in Examples.
Fig. 20 is a diagram showing final ranking of unknown waveform data obtained in Examples.
Fig. 21 is a diagram schematically illustrating a learning method of a neural network.
Fig. 22 is a diagram showing an example in which unknown waveform data similar to an ideal waveform is input to a trained neural network.
Fig. 23 is a diagram showing an example in which unknown waveform data dissimilar to the ideal waveform is input to the trained neural network.
Fig. 24 is a diagram schematically illustrating a learning method of an auto-encoder.
Fig. 25 is a diagram showing an example in which the unknown waveform data similar to the ideal waveform is input to a trained auto-encoder.
Fig. 26 is a diagram showing an example in which the unknown waveform data dissimilar to the ideal waveform is input to the trained auto-encoder.
Fig. 27 is a flowchart showing a determination process according to Modification Example.
Fig. 28 is a flowchart illustrating an example in which a filtering process is performed during execution of clustering.
Fig. 29 is a diagram showing an example of the filtering process.

DESCRIPTION OF THE PREFERRED EMBODIMENTS

Hereinafter, embodiments according to the technology of the present disclosure will be described with reference to the drawings.

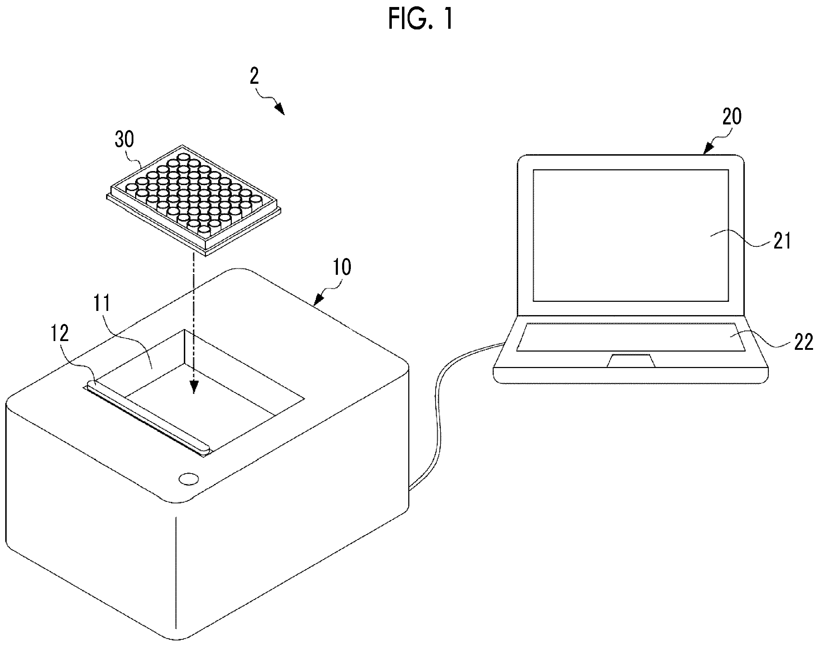

Fig. 1 schematically shows an electrical function evaluation system 2 that measures electrical activity of cells. The electrical function evaluation system 2 shown in Fig. 1 is configured of a cell culture apparatus 10 and an information processing apparatus 20. The cell culture apparatus 10 enables measurement of a waveform indicating an electrophysiological change of cells (for example, a myocardial waveform indicating pulsation of a myocardial cell) while culturing the cells. In addition, the cell culture apparatus 10 controls a culture environment (for example, a temperature and a carbon dioxide concentration).

An MEA plate 30 is used for culturing the cells. The cell culture apparatus 10 is provided with a culture chamber 11 that accommodates the MEA plate 30 therein. In addition, the cell culture apparatus 10 is provided with a slide-type lid 12 for opening and closing the culture chamber 11. The MEA plate 30 is attached to the culture chamber 11 in a state where the cells have been seeded. The culture chamber 11 functions as an incubator and enables the culture of the cells for a long period of time.

In the present embodiment, as the cells, myocardial cells produced from iPS cells are cultured by the cell culture apparatus 10. In addition, the cell culture apparatus 10 measures an extracellular potential representing a myocardial waveform of the myocardial cells seeded on the MEA plate 30 by a multipoint measurement method, and outputs waveform data obtained by the measurement to the information processing apparatus 20. The waveform data represents a pulse signal output by the myocardial cells.

The information processing apparatus 20 is configured of a general computer such as a personal computer. Software for analyzing the waveform data input from the cell culture apparatus 10 is installed in the information processing apparatus 20. The information processing apparatus 20 includes a display unit 21 and an input unit 22. The display unit 21 is a display device such as a liquid crystal display or an organic electro luminescence (EL) display. The input unit 22 is an input device such as a keyboard, a touch pad, or a mouse. The information processing apparatus 20 is connected to the cell culture apparatus 10 by wire or wirelessly. The display unit 21 and the input unit 22 may be configured as external devices connected to the information processing apparatus 20.

The information processing apparatus 20 calculates an extracellular potential duration (field potential duration (FPD)), an interspike interval (ISI), and the like based on the input waveform data. Since the FPD corresponds to a QT interval (time from a start of a Q wave to an end of a T wave) in an electrocardiogram, it is used as an index of arrhythmia. Prolongation of the QT interval indicates a potential for arrhythmia. A user can perform a toxicity evaluation for evaluating responsiveness of the cells to a drug based on the FPD or the like.

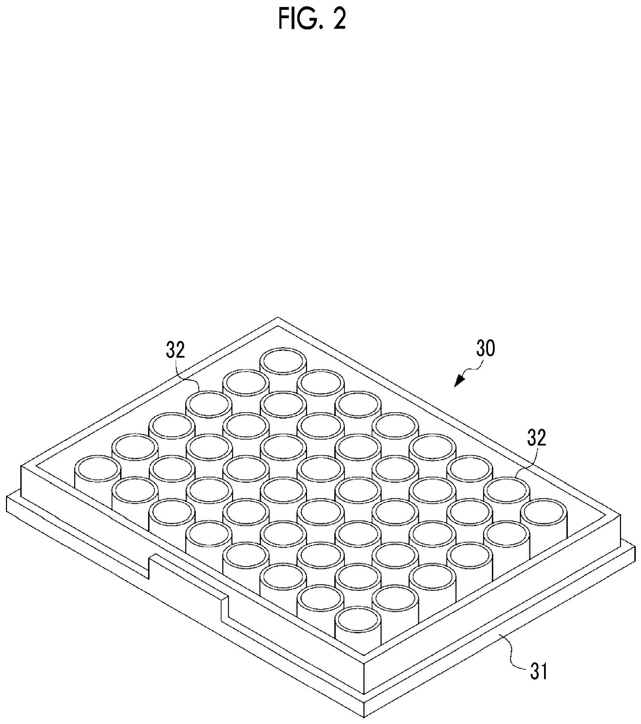

Fig. 2 shows an example of the MEA plate 30. The MEA plate 30 is a multi-well plate in which a plurality of culture wells (hereinafter, simply referred to as wells) 32 are arranged on a substrate 31. The MEA plate 30 shown in Fig. 2 has 48 wells 32. The number of the wells 32 provided in the MEA plate 30 is not limited to 48, and may be 24, 96, or the like.

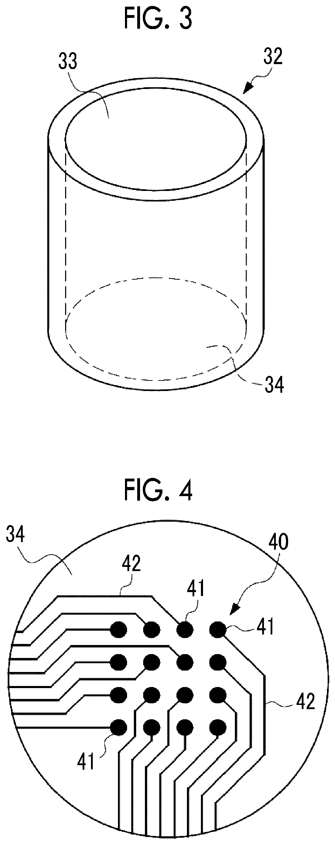

Fig. 3 shows an example of the well 32. The well 32 is a substantially cylindrical vessel having an opening 33 formed at a top. The cell is seeded to adhere to a bottom portion 34 of the well 32. The well 32 is filled with a culture solution containing a culture medium. A microelectrode array 40 (see Fig. 4) is embedded in the bottom portion 34 of the well 32.

Fig. 4 shows an example of the microelectrode array 40. The microelectrode array 40 has a plurality of electrodes 41. In the example shown in Fig. 4, the microelectrode array 40 has 16 microelectrodes (hereinafter, simply referred to as electrodes) 41 squarely arranged in $4 \times 4$. The electrodes 41 are exposed to the bottom portion 34 of the well 32 and come into contact with the seeded cells. Each of the electrodes 41 is connected to a potential measurement circuit 50, which will be described below, via a wiring line 42. Hereinafter, the electrode 41 may be denoted by as a channel CH. The 16 electrodes 41 are distinguished by being denoted by channels CH1 to CH16.

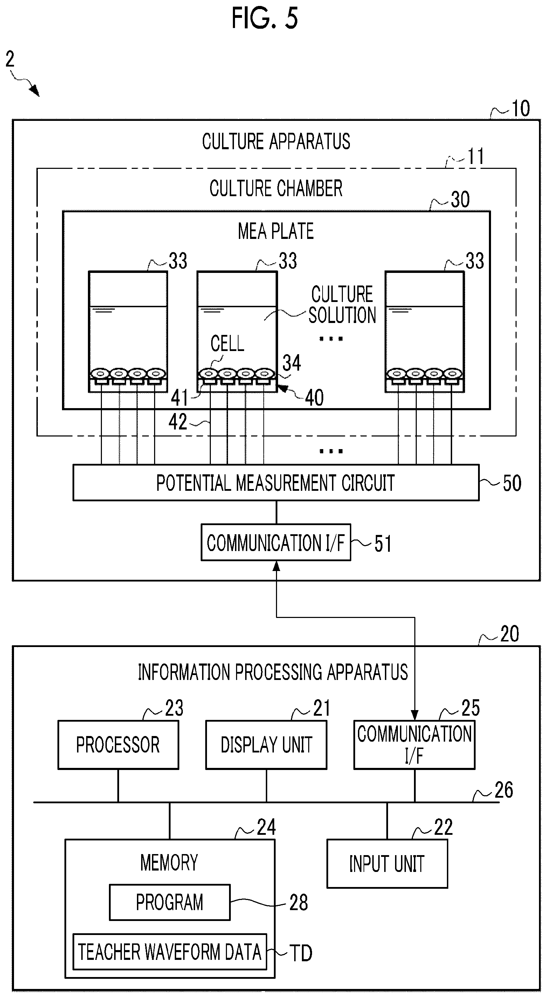

Fig. 5 shows an example of a hardware configuration of the electrical function evaluation system 2. The cell culture apparatus 10 includes a culture chamber 11, a potential measurement circuit 50, and a communication interface (I/F) 51. The potential measurement circuit 50 measures the extracellular potential of the myocardial cells cultured in the MEA plate 30 accommodated in the cell culture apparatus 10. Specifically, the potential measurement circuit 50 measures the extracellular potential via each electrode 41 of the microelectrode array 40 provided in each of the wells 32. That is, 16 myocardial waveforms are measured for each well 32 by the potential measurement circuit 50.

The potential measurement circuit 50 transmits the measured myocardial waveform to the information processing apparatus 20 as waveform data via the communication I/F 51. In a case in which the number of the wells 32 formed in the MEA plate 30 is 48 and the number of the microelectrode arrays 40 provided in one well 32 is 16, 768 pieces of the waveform data are transmitted from the potential measurement circuit 50 to the information processing apparatus 20.

The information processing apparatus 20 includes a processor 23, a memory 24, an input unit 22, a display unit 21, a communication I/F 25, a bus 26, and the like. The processor 23 is a computer that realizes various functions by reading out a program 28 and various types of data stored in the memory 24 and executing processing. The processor 23 is, for example, a central processing unit (CPU).

The memory 24 is a storage device that stores the program 28 and the various types of data in a case in which the processor 23 executes processing. The memory 24 includes, for example, a random access memory (RAM), a read only memory (ROM), a storage, or the like. The RAM is, for example, a volatile memory used as a work area or the like of the processor 23. The ROM is, for example, a non-volatile memory that holds the program 28 and the various types of data. The ROM is, for example, a flash memory. The storage is, for example, a large-capacity storage device such as a hard disk drive (HDD) or a solid state drive (SSD), and stores an operating system (OS), various types of data, and the like. The memory 24 may be configured as an external device connected to the information processing apparatus 20.

In addition, the memory 24 stores teacher waveform data TD. The teacher waveform data TD is waveform data to which determination results of a determination of superiority or inferiority indicating whether or not a waveform is close to a known ideal waveform are linked. The determination of the superiority or inferiority is performed by a sensory evaluation such as comparison with a known ideal waveform by a human. The ideal waveform is a waveform representing a healthy state (for example, a state without a disease such as arrhythmia) obtained by the past measurement.

Variations occur in the output waveforms of the plurality of electrodes 41 included in the microelectrode array 40 provided in the well 32. For the toxicity evaluation, it is necessary to use a waveform close to the ideal waveform. As will be described below in detail, the information processing apparatus 20 performs a process of selecting the electrode 41 from which a waveform with the highest similarity to the ideal waveform is output from the plurality of electrodes 41 included in the microelectrode array 40, as a target electrode of the toxicity evaluation. Such selection of the target electrode is also referred to as selection of a golden channel (hereinafter, referred to as GC). The information processing apparatus 20 performs the GC selection process using a plurality of waveform data transmitted from the cell culture apparatus 10 and a plurality of teacher waveform data TD stored in advance in the memory 24.

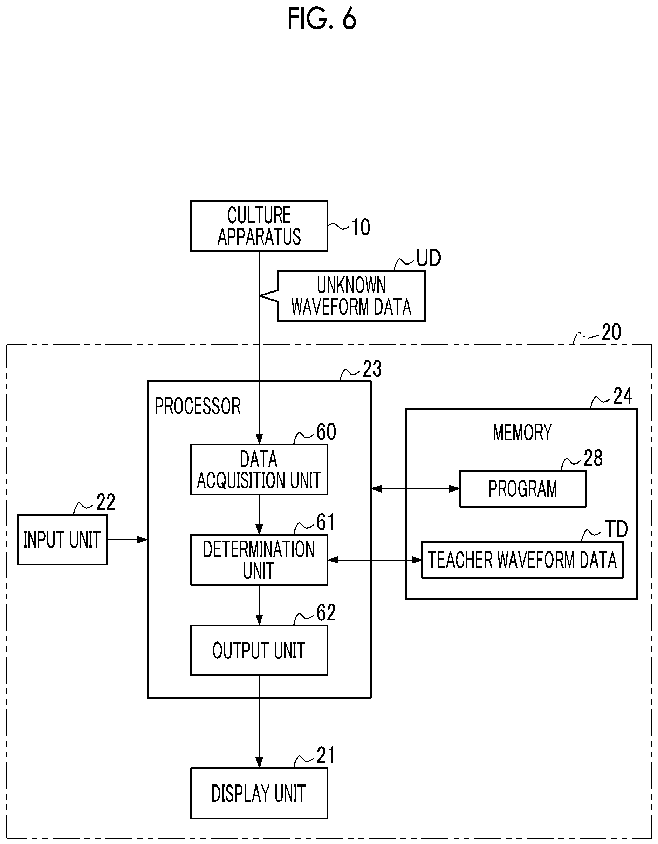

Fig. 6 shows an example of a functional configuration of the information processing apparatus 20. As for the functions shown in Fig. 6, the information processing apparatus 20 realizes various functions by executing the processing by the processor 23 based on the program 28. These various functions may be realized by hardware.

The processor 23 functions as a data acquisition unit 60, a determination unit 61, and an output unit 62. The data acquisition unit 60 performs an acquisition process of acquiring the waveform data transmitted from the cell culture apparatus 10. Since similarity of the waveform data transmitted from the cell culture apparatus 10 to the ideal waveform is unknown, the waveform data acquired by the data acquisition unit 60 from the cell culture apparatus 10 will be referred to as unknown waveform data UD below. In the unknown waveform data UD, the determination result of the superiority or inferiority based on the similarity to the ideal waveform is unknown.

The determination unit 61 performs a determination process of determining the superiority or inferiority with respect to the similarity to the ideal waveform for each of the plurality of unknown waveform data UD acquired by the data acquisition unit 60 based on the plurality of teacher waveform data TD stored in advance in the memory 24. As will be described below in detail, in the present embodiment, the determination unit 61 determines the superiority or inferiority by using a method of clustering that is a kind of machine learning algorithm.

The output unit 62 performs an output process of outputting the superiority or inferiority of the plurality of unknown waveform data UD determined by the determination unit 61 in a comparable manner. For example, the output unit 62 causes the display unit 21 to display the superiority or inferiority of the plurality of unknown waveform data UD in a comparable manner.

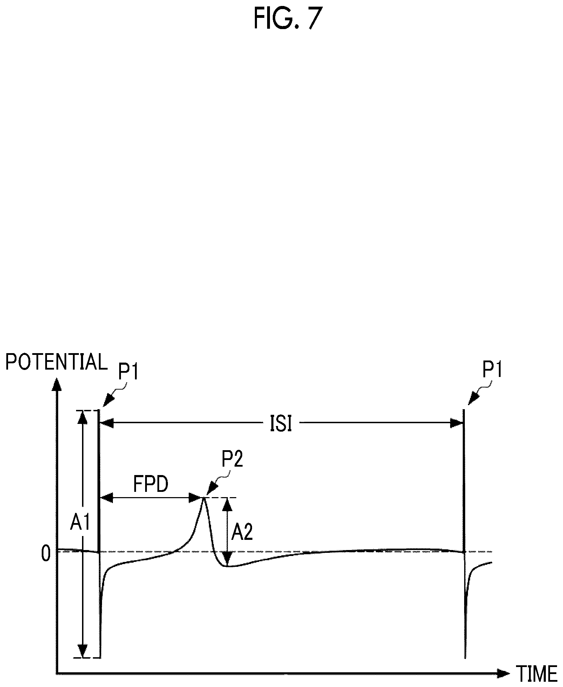

Fig. 7 shows an example of a myocardial waveform. The myocardial waveform shown in Fig. 7 is a measurement waveform of an extracellular potential. In Fig. 7, the FPD is the above-mentioned extracellular potential duration, and the ISI is the interspike interval. A1 is an amplitude of a first peak P1, and A2 is an amplitude of a second peak P2. These values vary depending on a state of the cell or various factors in measurement.

The superiority or inferiority of the teacher waveform data TD is determined by a human evaluation using values of FPD, ISI, maximum potential of P1 (hereinafter, referred to as P1max), minimum potential of P1 (hereinafter, referred to as P1min), and A2, and the overall shape of the waveform as evaluation criteria. For example, a waveform that satisfies Equations (1) to (4) and satisfies an evaluation criterion that the overall shape is close to the ideal waveform is determined as being "superior" (that is, GC), and a waveform that does not satisfy the evaluation criterion is determined as being "inferior".

$$P1max \geq 200\ \mu V \ ... \ (1)$$

$$P1min \leq -200\ \mu V \ ... \ (2)$$

$$A2 \geq 15\ \mu V \ ... \ (3)$$

$$FPDcF \geq 340\ msec \ ... \ (4)$$

Here, FPDcF is a value obtained by dividing FPD by $ISI^{1/3}$.

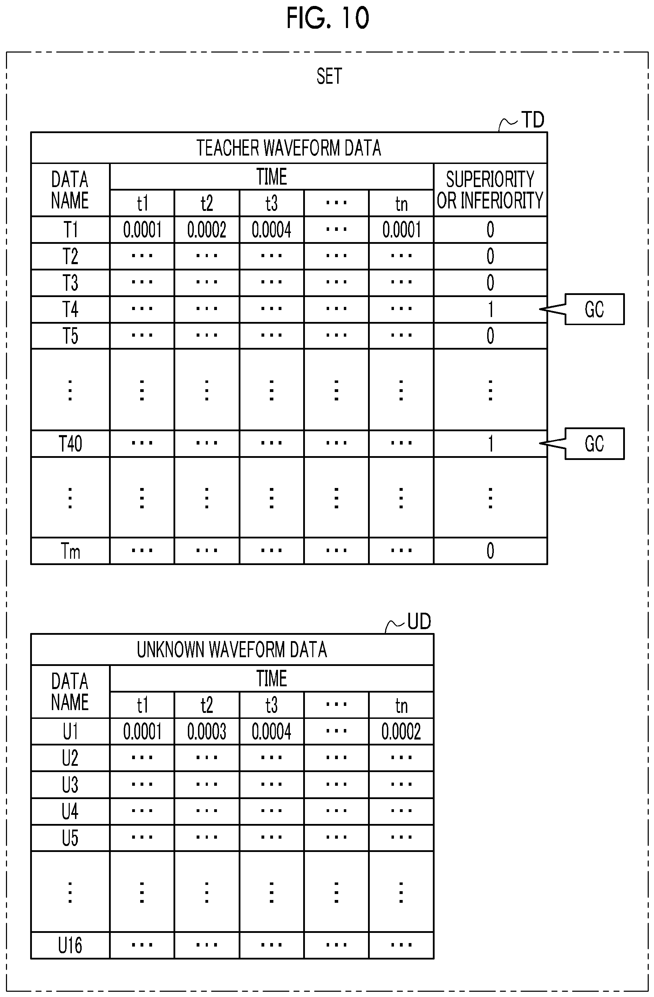

The above determination result is added to the teacher waveform data TD (see Fig. 10).

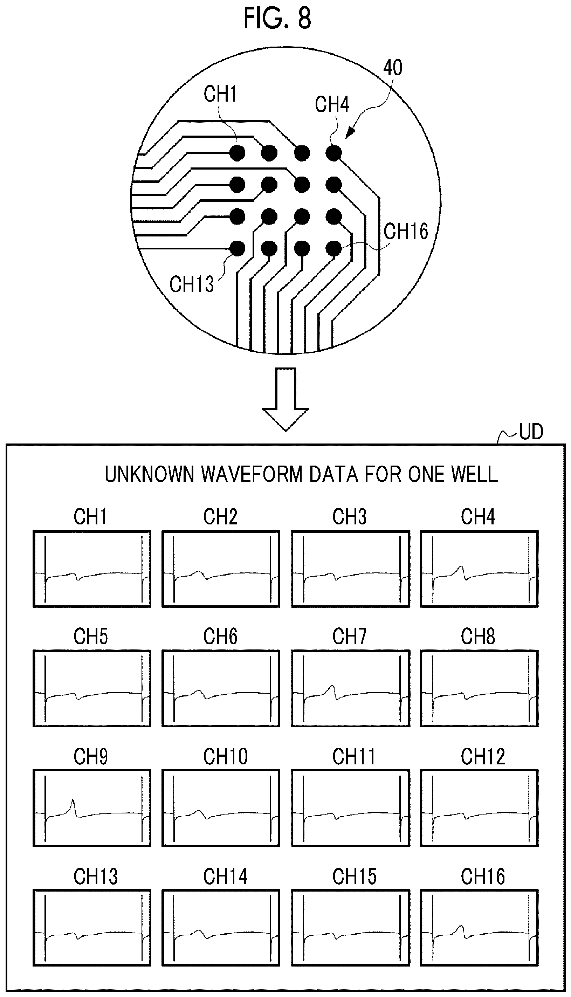

Fig. 8 shows an example of the unknown waveform data UD output from the plurality of electrodes 41 included in the microelectrode array 40. Fig. 8 shows the unknown waveform data UD corresponding to each of the channels CH1 to CH16. The determination unit 61 determines the superiority or inferiority for each unknown waveform data UD by a determination process described below, and selects the electrode 41 from which a waveform closest to the ideal waveform is obtained, as the target electrode (that is, GC).

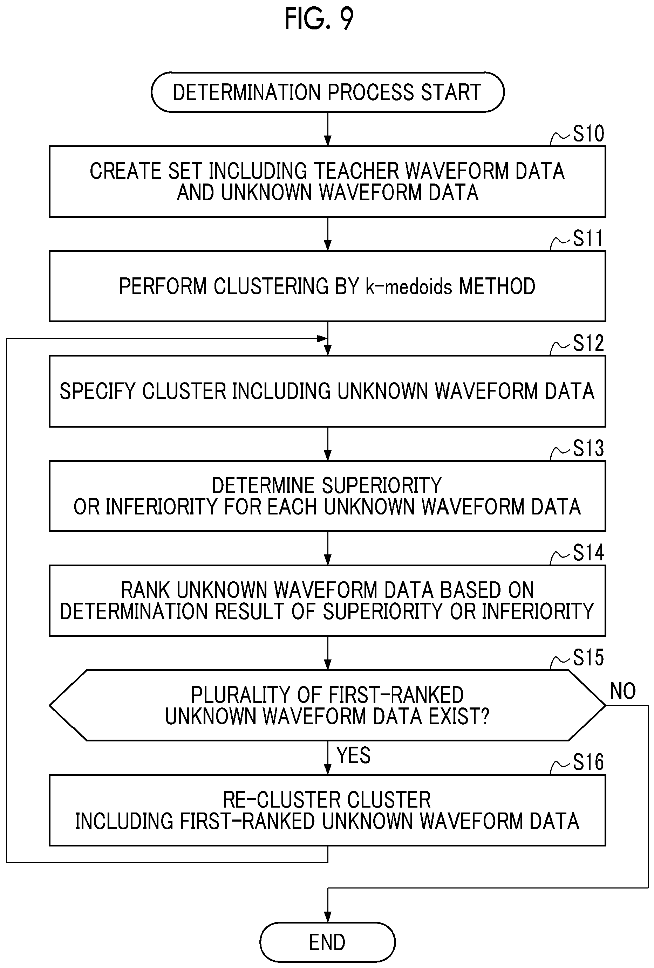

Fig. 9 shows an example of the determination process by the determination unit 61. The example of the determination process will be described with reference to a flowchart shown in Fig. 9. First, the determination unit 61 creates a set including the plurality of unknown waveform data UD acquired by the data acquisition unit 60 and the plurality of teacher waveform data TD stored in the memory 24 (step S10).

Fig. 10 is an example of the set created in step S10. The unknown waveform data UD and the teacher waveform data TD are time-series data in a period (see Fig. 7) including one interspike interval (ISI). t1 to tn represent n distinct times. For example, n = 7250. T1 to Tm are data names for identifying m pieces of the teacher waveform data TD. For example, m = 1044. U1 to U16 are data names for identifying 16 pieces of the unknown waveform data UD corresponding to the channels CH1 to CH16.

The teacher waveform data TD is linked with the determination result of the superiority or inferiority based on a human evaluation. "1" indicates that the waveform is determined as being similar to the ideal waveform (that is, GC). "0" indicates that the waveform is determined as being dissimilar to the ideal waveform (that is, not GC). That is, "1" indicates that the determination result of the superiority or inferiority based on the similarity to the ideal waveform is "superior determination", and "0" indicates that the determination result of the superiority or inferiority based on the similarity to the ideal waveform is "inferior determination".

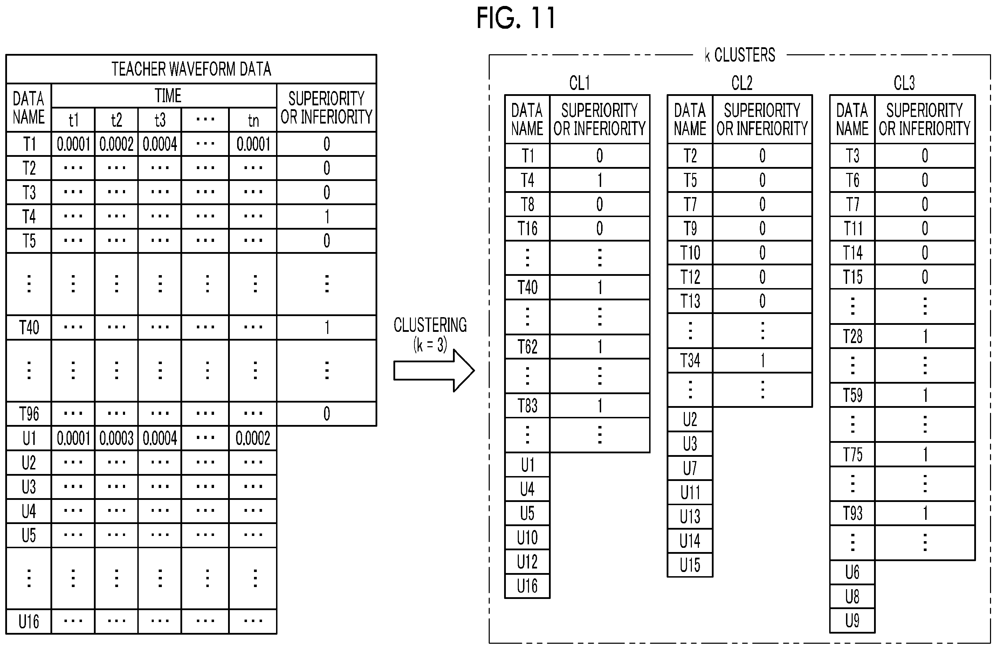

Next, the determination unit 61 combines the plurality of unknown waveform data UD and the plurality of teacher waveform data TD included in the created set, and performs clustering by a k-medoids method (step S11). As an example, as shown in Fig. 11, the determination unit 61 performs clustering with, for example, the number of clusters k being 3. Accordingly, the unknown waveform data UD and the teacher waveform data TD are distributed to any one of three clusters CL1 to CL3.

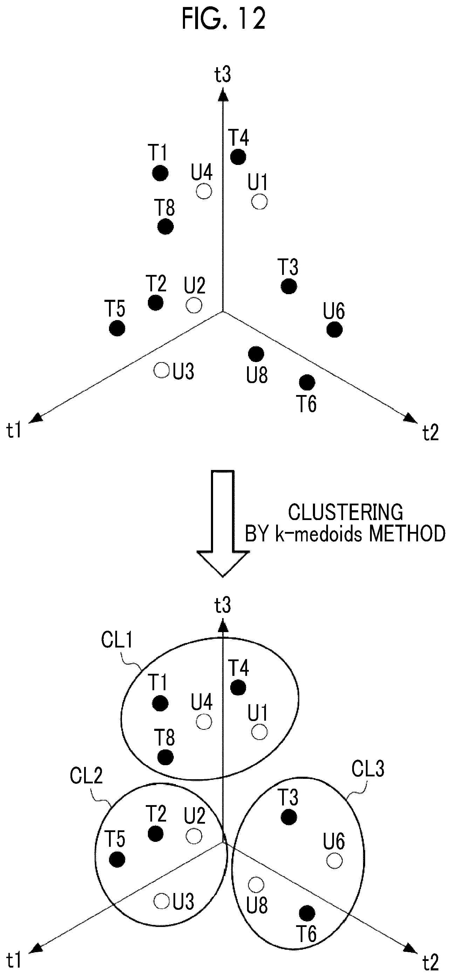

Fig. 12 is a diagram illustrating a method of the clustering. As shown in Fig. 12, the clustering is performed by treating each of the plurality of unknown waveform data UD and the plurality of teacher waveform data TD as points plotted in an n-dimensional space. n axes representing the n-dimensional space are t1 to tn described above. Fig. 12 is a diagram in which the unknown waveform data UD and the teacher waveform data TD are plotted in a three-dimensional space with t1 to t3 as the axes, for the purpose of simplifying the description.

A known k-medoids method is used for the clustering. In the k-medoids method, first, k points are randomly selected as medoids in the n-dimensional space. Next, each point is assigned to the closest medoid cluster. Then, in each cluster, a new medoid is set such that a total of distances to all the other points in the cluster is minimized. After that, the processing is repeated until there is no change in the medoid. In a case of the present embodiment, since k = 3, three clusters CL1 to CL3 are generated. It is also possible to use a k-means method instead of the k-medoids method. In the k-means method, the centroid of a point in the cluster is calculated, and the processing is repeated until there is no change in the centroid.

Next, the determination unit 61 specifies a cluster including the unknown waveform data UD from the plurality of clusters generated by the clustering (step S12). In the example shown in Fig. 11, since the unknown waveform data UD is included in all the clusters CL1 to CL3, all the clusters CL1 to CL3 are specified as the cluster including the unknown waveform data UD.

Next, the determination unit 61 determines the superiority or inferiority for each of the unknown waveform data UD included in the clusters specified in step S12 (step S13). Specifically, the determination unit 61 determines the superiority or inferiority by obtaining a probability (hereinafter, referred to as a score SC) that the unknown waveform data UD is superior determination "1" for each of the specified clusters. More specifically, the determination unit 61 obtains the score SC based on the number N1 of the teacher waveform data with the superior determination "1" and the number NT of the teacher waveform data with the superior determination "1" and the inferior determination "0", for each of the specified clusters. NT corresponds to the number of the teacher waveform data TD included in the cluster.

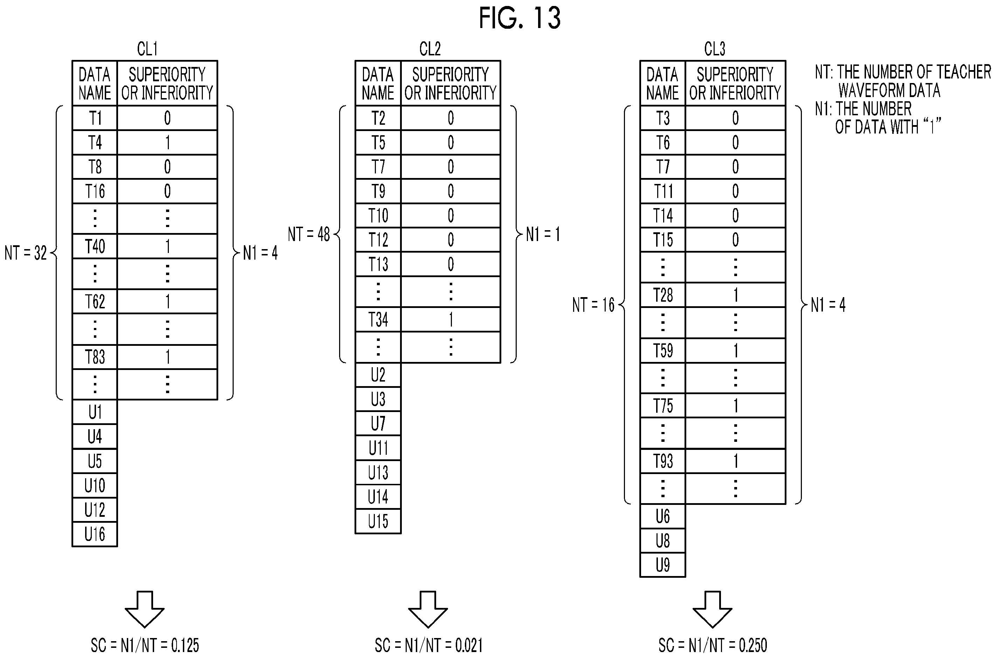

As shown in Fig. 13, the score SC is calculated by dividing the number N1 of the teacher waveform data with the superior determination "1" by the number NT of the teacher waveform data with the superior determination "1" and the inferior determination "0" (that is, N1/NT). In the example shown in Fig. 13, the score SC of the cluster CL1 is 0.125, the score SC of the cluster CL2 is 0.021, and the score SC of the cluster CL3 is 0.250. Therefore, the unknown waveform data U6, U8, and U9 included in the cluster CL3 have the highest score SC.

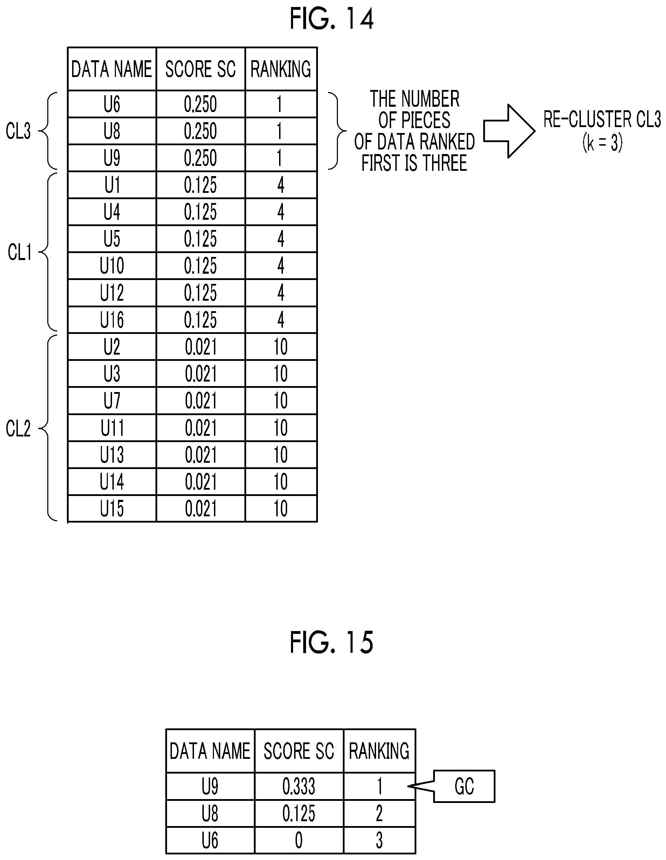

Next, the determination unit 61 ranks the unknown waveform data UD based on the determination result of the superiority or inferiority (step S14). Fig. 14 shows an example in which the unknown waveform data UD included in the clusters CL1 to CL3 are ranked in descending order of the score SC representing the determination result of the superiority or inferiority based on the score SC. In the example shown in Fig. 14, the unknown waveform data U6, U8, and U9 included in the cluster CL3 are ranked first.

Next, the determination unit 61 determines whether or not a plurality of the first-ranked unknown waveform data UD exist (step S15). In a case in which the determination unit 61 determines that a plurality of the first-ranked unknown waveform data UD exist (step S15: YES), the determination unit 61 re-clusters the cluster including the first-ranked unknown waveform data UD (step S16). A method of the clustering is the same as in step S11. In the example shown in Fig. 14, since three unknown waveform data U6, U8, and U9 are ranked first, the cluster CL3 (see Fig. 11) including them is re-clustered.

After step S16, the determination unit 61 returns the processing to step S12. After that, the determination unit 61 executes each of the processes of steps S12 to S15 on a plurality of subclusters obtained by performing the clustering on the cluster CL3. In a case in which the determination unit 61 determines that a plurality of the first-ranked unknown waveform data UD do not exist (that is, there is only one first-ranked unknown waveform data UD) (step S15: NO), a determination process is ended.

Fig. 15 shows an example in which the unknown waveform data U6, U8, and U9 are ranked as a result of executing each of the processes of steps S12 to S15 after performing the clustering on the cluster CL3. In the example shown in Fig. 15, only the unknown waveform data U9 is ranked first. That is, the electrode 41 (that is, the channel CH9) from which the unknown waveform data U9 is obtained is selected as the target electrode (GC) for the toxicity evaluation.

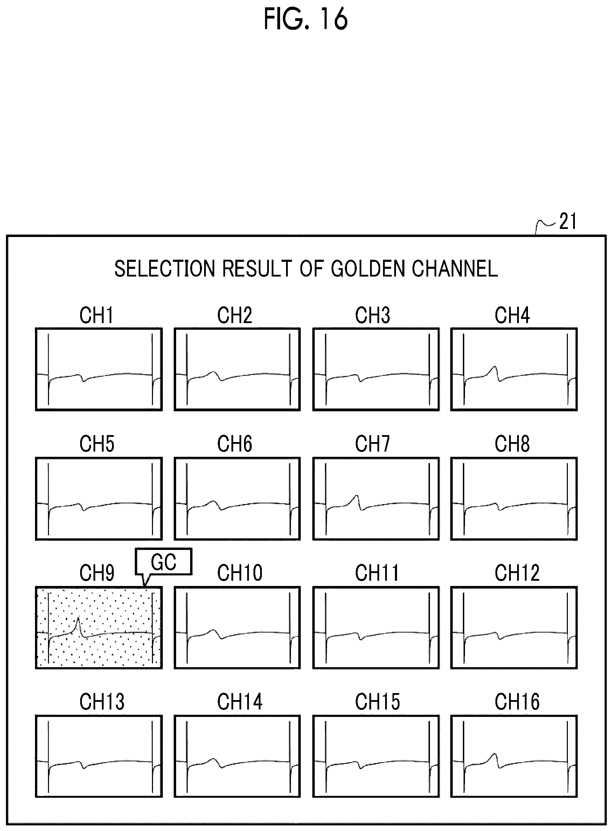

The output unit 62 makes it possible to compare the superiority or inferiority of the plurality of unknown waveform data UD by, for example, causing the display unit 21 to display the table shown in Fig. 15. In addition, as shown in Fig. 16, the output unit 62 may display the GC selected from the channels CH1 to CH16 in a specifiable manner. The display example shown in Fig. 16 is an example of "an aspect in which superiority or inferiority of a plurality of unknown waveform data is output in a comparable manner" according to the technology of the present disclosure.

As described above, according to the technology of the present disclosure, the determination of the superiority or inferiority is performed for each of the plurality of unknown waveform data based on the plurality of teacher waveform data to which the determination result of the superiority or inferiority is linked, and the superiority or inferiority of the plurality of unknown waveform data is output in a comparable manner. Accordingly, it is possible to select a waveform close to the ideal waveform with high accuracy in a short time.

In addition, it is possible to evaluate a drug based on the determination information of the superiority or inferiority output from the information processing apparatus 20. Among the plurality of unknown waveform data, the unknown waveform data with a high rank of the superiority or inferiority determined by the information processing apparatus 20 may be used for the drug evaluation.

[Examples]

Hereinafter, examples of the determination process of the superiority or inferiority will be described.

In this example, a MAESTRO768PRO manufactured by Axion BioSystems was used as the cell culture apparatus 10, and a multi-well plate provided with 24 wells 32 was used as the MEA plate 30. Then, the myocardial waveform was measured while culturing the myocardial cells in each well 32 of the cell culture apparatus 10. Waveform data was generated by extracting data of 250 points in a negative direction and 7000 points in a positive direction from a point with the highest voltage as a zero point on a time axis of waveform data measured between 150 seconds and 135 seconds after a start of the measurement. This waveform data represents a myocardial waveform including one interspike interval.

Based on the waveform data acquired by the cell culture apparatus 10, the teacher waveform data TD for three plates were prepared, in which the determination results of the superiority or inferiority were linked with each other by evaluation by a human in the past. That is, the number of the prepared teacher waveform data TD is 1152. In addition, based on the waveform data acquired by the cell culture apparatus 10, the unknown waveform data UD for one well for newly determining the superiority or inferiority was prepared. That is, the number of the prepared unknown waveform data UD is 16.

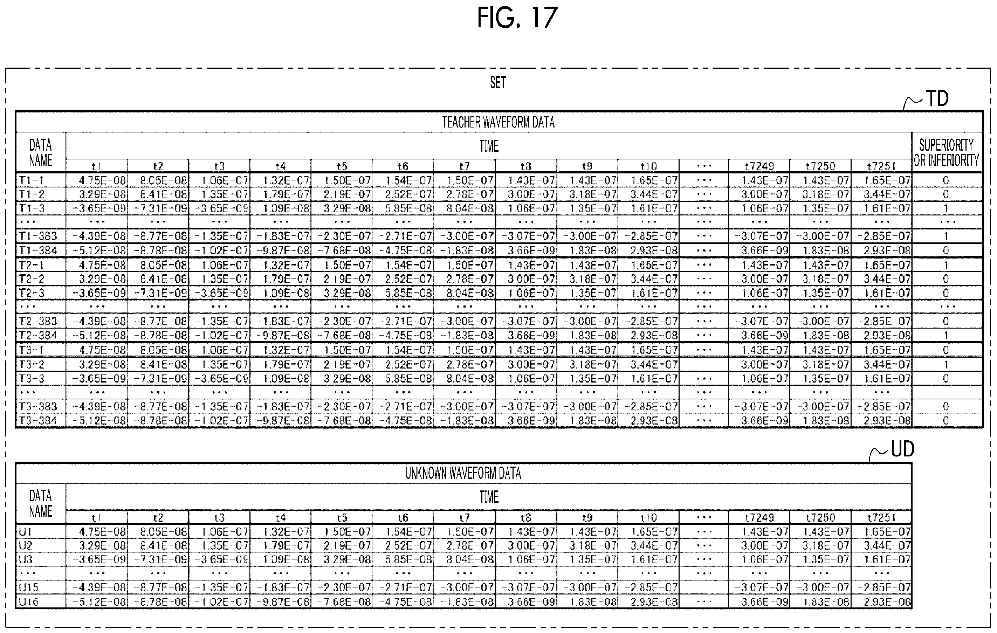

Fig. 17 shows a set including the plurality of teacher waveform data TD and the plurality of unknown waveform data UD prepared in this example. A data name of the teacher waveform data TD represents a plate number and a serial number of the waveform data in the plate. For example, T2-383 represents that the plate number is 2 and the serial number is 383.

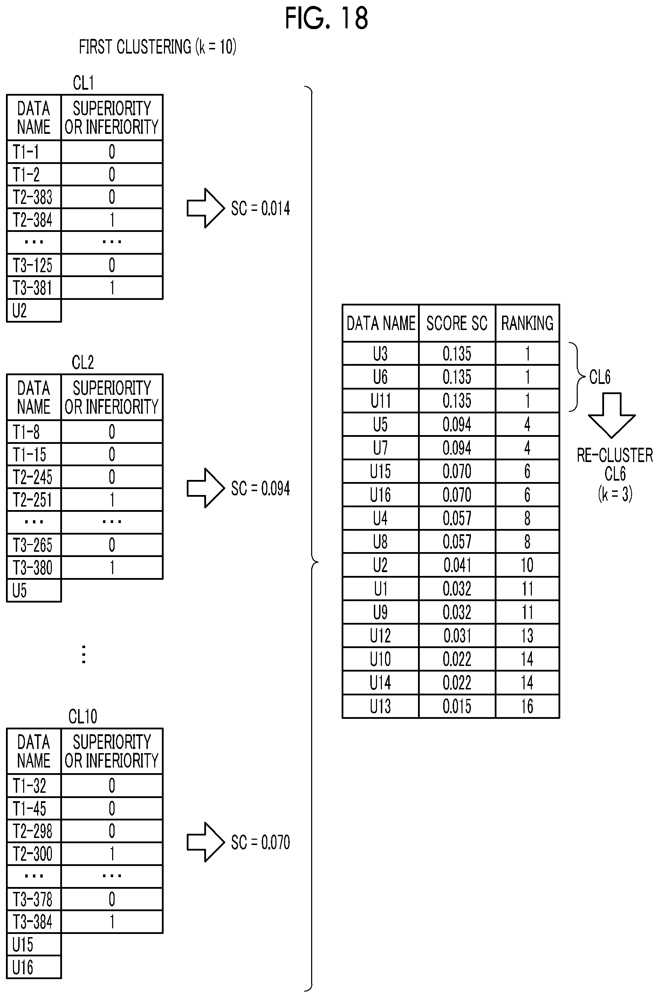

Next, the plurality of unknown waveform data UD and the plurality of teacher waveform data TD included in the set were combined, and clustering (first clustering) was performed by the k-medoids method using MATLAB (registered trademark), which is numerical analysis software manufactured by MathWorks (registered trademark). Here, k = 10. Fig. 18 shows results of clustering the plurality of unknown waveform data UD and the plurality of teacher waveform data TD included in the set shown in Fig. 17. Ten clusters generated by the clustering are numbered 1 to 10 without duplication.

Next, a score SC was calculated for each of the clusters including the unknown waveform data UD, and, based on the calculated score SC, the unknown waveform data UD were ranked in descending order of the score SC. Fig. 18 shows a result of ranking the unknown waveform data UD. Three pieces of the unknown waveform data U3, U6, and U11 were ranked first. Therefore, the cluster CL6 including the first-ranked unknown waveform data U3, U6, and U11 was subjected to re-clustering (second clustering). Here, since the number of the first-ranked unknown waveform data UD is 3, k = 3.

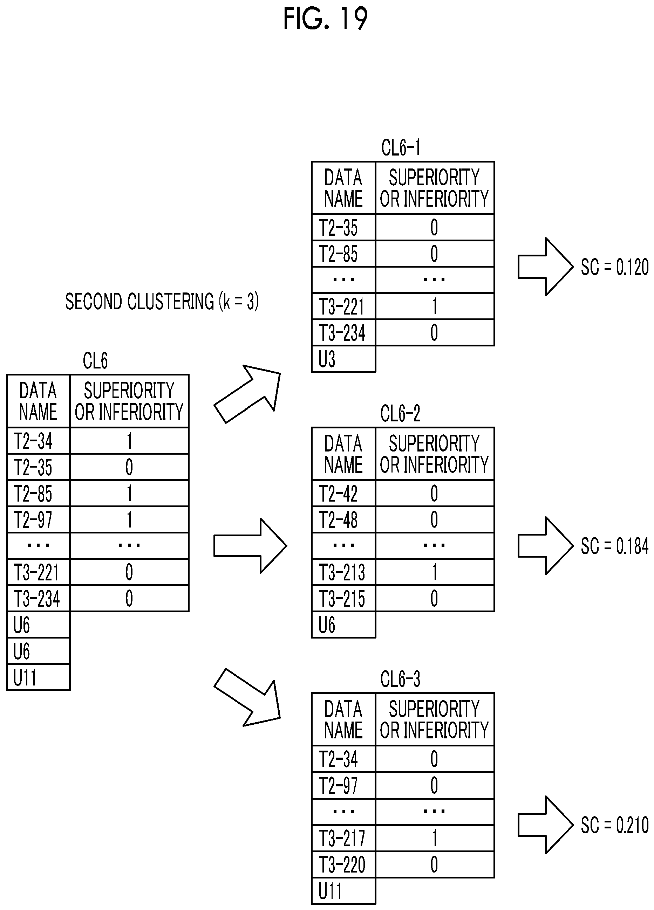

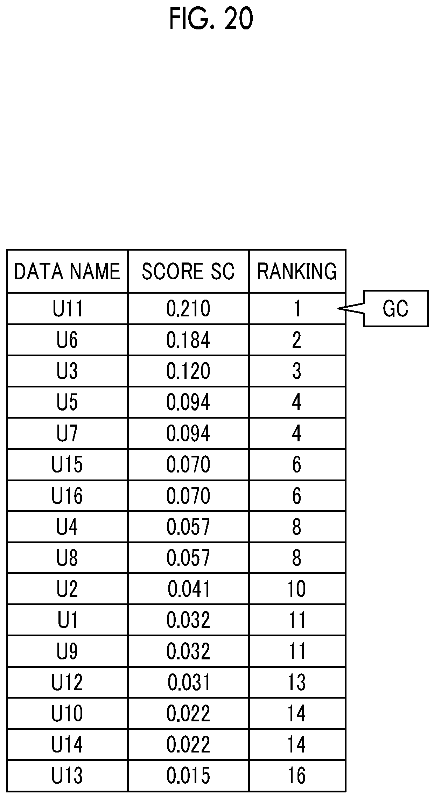

Fig. 19 shows a result of re-clustering the cluster CL6. As shown in Fig. 19, a score SC was calculated for each of three subclusters CL6-1, CL6-2, and CL6-3 generated by re-clustering the cluster CL6. Then, as shown in Fig. 20, the unknown waveform data U3, U6, and U11 included in the cluster CL6 were ranked based on the score SC. As a result, the score SC of the unknown waveform data U11 was the highest, and only the unknown waveform data U11 was ranked first. Therefore, the channel CH 16 was selected as the GC.

As described above, it was confirmed that, by using the method of the clustering, it is possible to select a waveform close to the ideal waveform from the plurality of unknown waveform data with high accuracy in a short time.

[Modification Example]

Next, various modification examples of the above embodiment will be described.

In the above-described embodiment, the teacher waveform data TD is linked with binary data of "1" or "0" as the determination result of the superiority or inferiority, but data of three or more values may be linked with the teacher waveform data TD. That is, the determination result of the superiority or inferiority is not limited to the one represented by the binary value, and may be represented by the three or more values.

In the above-described embodiment, the determination unit 61 determines the superiority or inferiority of the unknown waveform data UD by the clustering, but the determination unit 61 is not limited to the clustering, and may determine the superiority or inferiority of the unknown waveform data UD using a neural network that has been trained through machine learning.

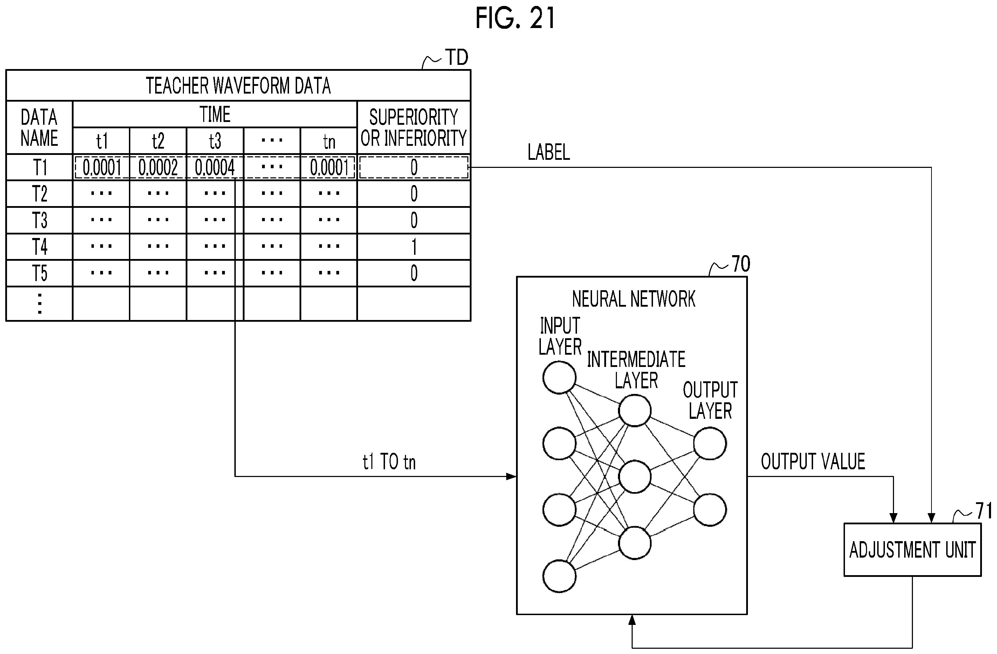

Fig. 21 schematically illustrates a learning method of a neural network 70 used by the determination unit 61. In a learning phase, the neural network 70 is trained using time-series data t1 to tn of the teacher waveform data TD as explanatory variables and the determination result of the superiority or inferiority linked with the teacher waveform data TD as a response variable. The determination result of the superiority or inferiority is a "label" in so-called labeled machine learning.

In the learning phase, in a case in which the time-series data t1 to tn are input to an input layer of the neural network 70, an output value from an output layer is input to an adjustment unit 71. In addition, the determination result of the superiority or inferiority as a label is input to the adjustment unit 71. The adjustment unit 71 compares the output value with the inferior determination result, and adjusts the weight and the bias of the neural network 70 based on a difference between the two values.

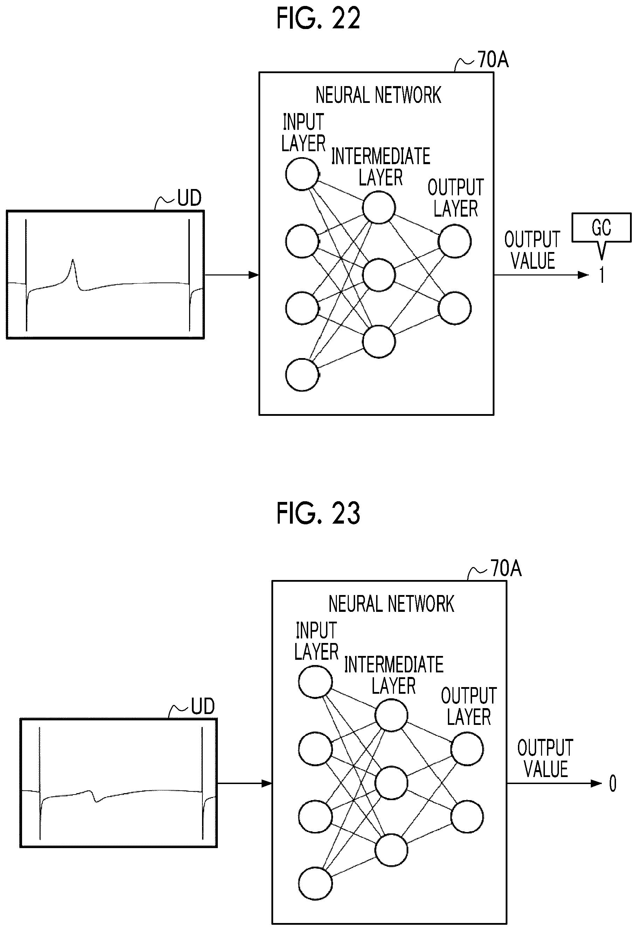

As shown in Figs. 22 and 23, the determination unit 61 determines the superiority or inferiority of the unknown waveform data UD using a trained neural network 70A that has been trained through machine learning using the plurality of teacher waveform data TD. As shown in Fig. 22, in a case in which the unknown waveform data UD similar to the ideal waveform is input to the neural network 70A, "1" is output as the output value. The fact that the output value is "1" indicates that the corresponding electrode 41 is GC. On the other hand, as shown in Fig. 23, in a case in which the unknown waveform data UD dissimilar to the ideal waveform is input to the neural network 70A, "0" is output as the output value. The fact that the output value is "0" indicates that the corresponding electrode 41 is not GC.

In this modification example as well, the teacher waveform data TD used for training the neural network 70 is linked with binary data of "1" or "0" as the determination result of the superiority or inferiority, but data of three or more values may be linked with the teacher waveform data TD. That is, the determination result of the superiority or inferiority is not limited to the one represented by the binary value, and may be represented by the three or more values. In this case, the output value from the neural network 70 is represented by three or more values.

In addition, the determination unit 61 may determine the superiority or inferiority of the unknown waveform data UD using an auto-encoder that has been trained through machine learning.

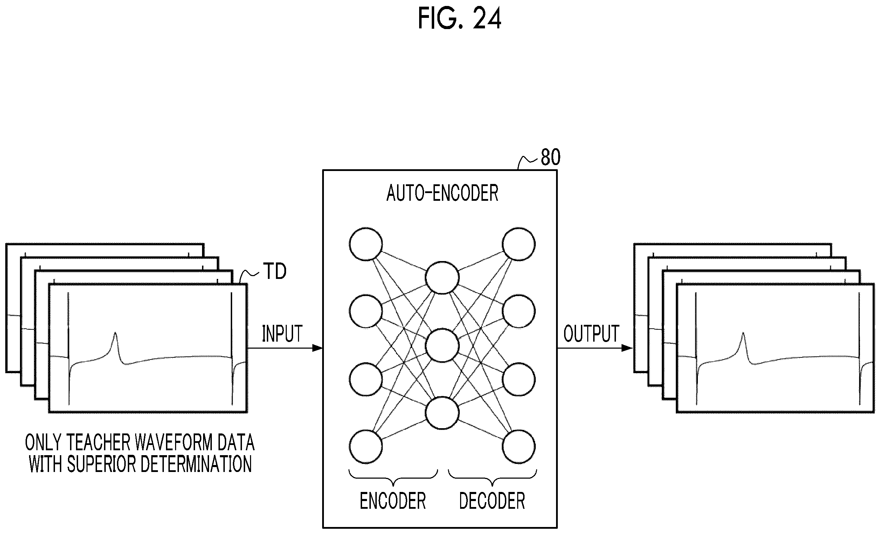

Fig. 24 schematically describes a learning method of an auto-encoder 80 used by the determination unit 61. The auto-encoder 80 includes an encoder and a decoder. The auto-encoder 80 extracts a feature amount by reducing a dimension of input data by the encoder, and restores and outputs the input data based on the extracted feature amount by the decoder. In a learning phase of the auto-encoder 80, the edge weights are adjusted such that the input and output match. Through this learning, important information necessary for restoration is extracted from the data, and a network for efficiently restoring the original data is formed.

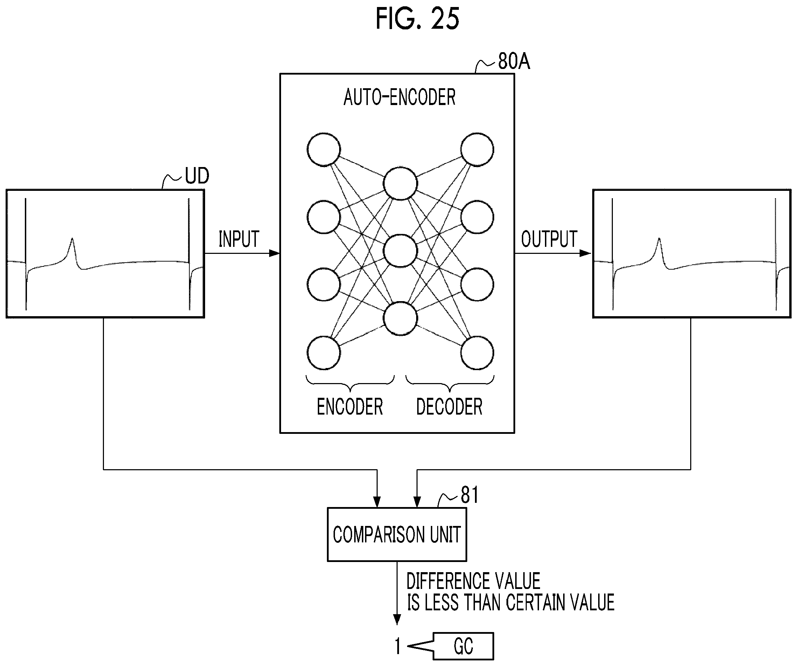

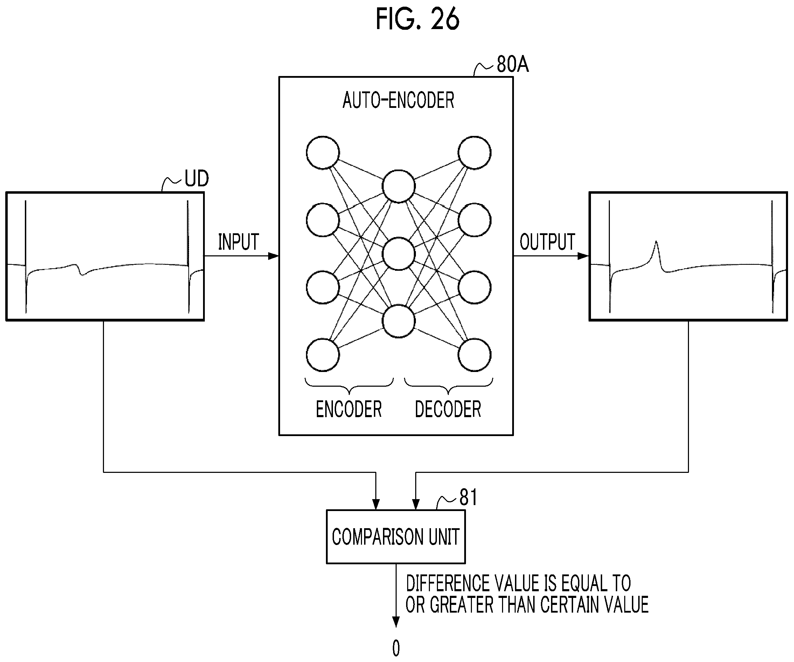

As shown in Fig. 24, in the learning phase, the auto-encoder 80 is trained using only the teacher waveform data TD with the superior determination. Accordingly, a trained auto-encoder 80A (see Figs. 25 and 26) outputs a waveform similar to the ideal waveform even in a case in which a waveform dissimilar to the ideal waveform is input. Therefore, similarity of the input waveform to the ideal waveform can be obtained by comparing the input waveform and the output waveform of the trained auto-encoder 80A. The determination unit 61 inputs the unknown waveform data UD to an encoder of the trained auto-encoder 80A, and then determines the superiority or inferiority of the unknown waveform data UD based on a difference between the waveform data restored by a decoder and the unknown waveform data UD input to the encoder.

Specifically, as shown in Figs. 25 and 26, the determination unit 61 determines the superiority or inferiority of the unknown waveform data UD by using the trained auto-encoder 80A that has been trained through machine learning using the plurality of teacher waveform data TD to which the determination result with the superior determination is linked, and a comparison unit 81 that compares the input and output data. The comparison unit 81 calculates a difference value of the input and output data, outputs "1" in a case in which the difference value is less than a certain value, and outputs "0" in a case in which the difference value is equal to or greater than a certain value.

As shown in Fig. 25, in a case in which the unknown waveform data UD similar to the ideal waveform is input to the auto-encoder 80A, the auto-encoder 80A outputs waveform data similar to the ideal waveform. In this case, the comparison unit 81 outputs "1". The fact that the output value from the comparison unit 81 is "1" indicates that the corresponding electrode 41 is GC. On the other hand, as shown in Fig. 26, even in a case in which the unknown waveform data UD dissimilar to the ideal waveform is input to the auto-encoder 80A, the auto-encoder 80A outputs waveform data similar to the ideal waveform. In this case, the comparison unit 81 outputs "0". The fact that the output value from the comparison unit 81 is "0" indicates that the corresponding electrode 41 is not GC.

In this modification example, the comparison unit 81 outputs binary data of "1" or "0" depending on the difference value, but the present invention is not limited to this, and may be configured to output data of three or more values.

Next, a modification example of the clustering will be described. The ranking of the unknown waveform data UD by the clustering described in the above-described embodiment is performed based on the similarity of the shape with the ideal waveform. In order to improve an accuracy in a discrimination of minute differences in peak values in the waveform, it is preferable to perform a filtering process of excluding unknown waveform data UD that does not satisfy the evaluation criteria from the plurality of unknown waveform data UD to be determined before executing the clustering, using the evaluation criteria represented by Equations (1) to (4).

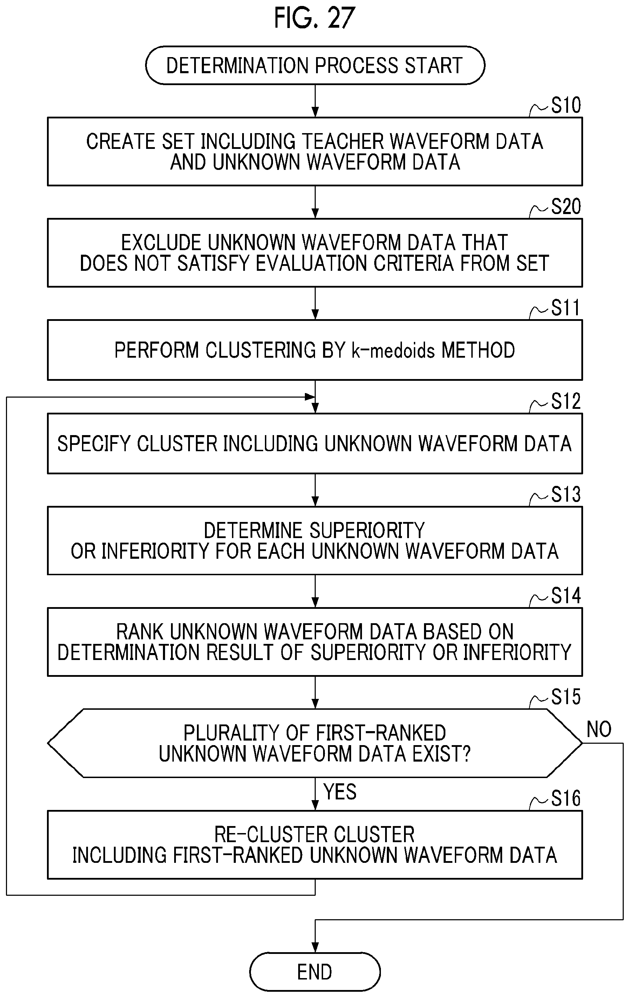

Fig. 27 shows a determination process according to the modification example. As shown in Fig. 27, in this modification example, the determination unit 61 executes step S10 of creating a set (see Fig. 10) including the plurality of unknown waveform data UD and the plurality of teacher waveform data TD, and then performs a process of excluding the unknown waveform data UD that does not satisfy the evaluation criteria from the set (step S20). Specifically, values of P1max, P1min, A2, and FPDcF are measured for each of the plurality of unknown waveform data UD, and unknown waveform data UD that does not satisfy at least one of Equations (1) to (4) is excluded from the set and excluded from the clustering. Steps S11 to S16 are the same as the processes described in the above-described embodiment.

In this way, by performing the filtering process of excluding unknown waveform data UD that does not satisfy the evaluation criteria before executing the clustering, the unknown waveform data UD that does not satisfy the evaluation criteria is prevented from being ranked higher by the clustering. Accordingly, the accuracy of the determination process by the determination unit 61 is improved.

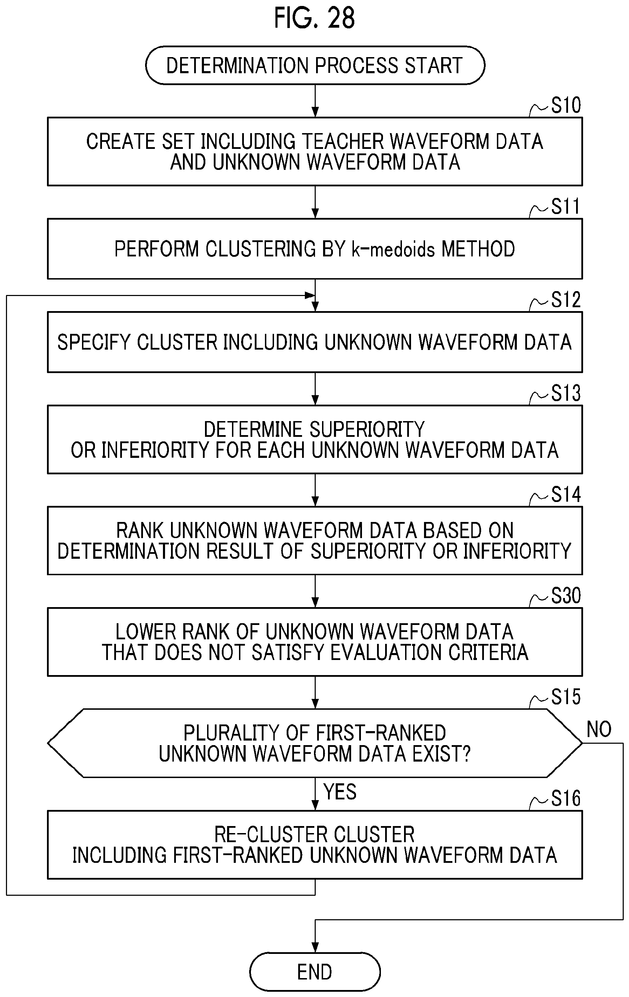

In the modification example shown in Fig. 27, the filtering process is executed before the clustering, but the filtering process may be performed during the execution of the clustering. For example, as shown in Fig. 28, the determination unit 61 executes step S14 of ranking the plurality of unknown waveform data UD based on the score SC, and then performs a process of lowering a rank of the unknown waveform data UD that does not satisfy the evaluation criteria in terms of the superiority or inferiority (step S30). A method of specifying the unknown waveform data UD that does not satisfy the evaluation criteria is the same as described above.

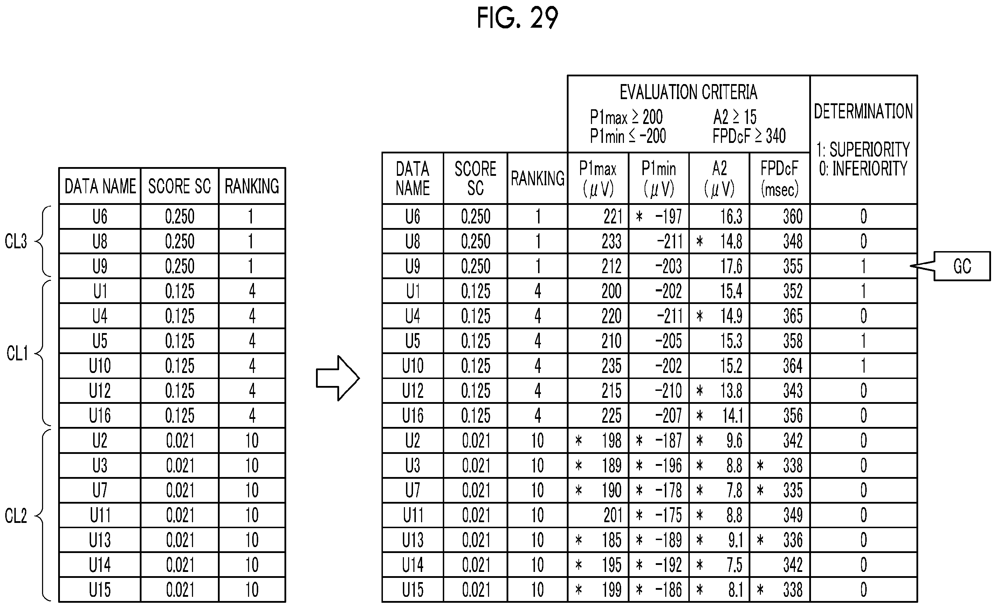

For example, as shown in Fig. 29, the determination unit 61 performs the filtering process on the plurality of unknown waveform data UD. The determination unit 61 determines the unknown waveform data UD that does not satisfy the evaluation criteria as an inferior determination and determining the unknown waveform data UD that satisfies the evaluation criteria as a superior determination, and lowers a rank of the unknown waveform data UD with the inferior determination (for example, lowers the rank to the lowest). In Fig. 29, an asterisk is added to a value that does not satisfy the evaluation criteria.

In the example shown in Fig. 29, as a result of the clustering, the unknown waveform data U6, U8, and U9 are all ranked first with the same rate, but the unknown waveform data U6 does not satisfy the evaluation criteria with respect to the value of P1min, and the unknown waveform data U8 does not satisfy the evaluation criteria with respect to A2. Therefore, in this example, the unknown waveform data U6 and U8 are determined as being inferior, and the rank thereof is lowered. As a result, only the unknown waveform data U9 is ranked first, and the electrode 41 (that is, the channel CH9) from which the unknown waveform data U9 is obtained is selected as the target electrode (GC) for the toxicity evaluation.

The filtering process in step S30 is not limited to immediately after step S14, and may be executed after it is determined in step S15 that a plurality of the first-ranked unknown waveform data UD exist. In this case, the filtering process may be performed only on the plurality of first-ranked unknown waveform data UD.

The evaluation criteria used in the filtering process are not limited to Equations (1) to (4) and can be appropriately changed.

In the above-described embodiment, the myocardial cell is used as the cell, but it is also possible to use a cell such as a nerve cell instead of the myocardial cell.

In the above-described embodiment, for example, a hardware structure of a processing unit that executes various kinds of processing, such as the data acquisition unit 60, the determination unit 61, and the output unit 62, is various processors as shown below.

Various processors include a CPU, a programmable logic device (PLD), a dedicated electric circuit, and the like. As is well known, the CPU is a general-purpose processor that executes software (program) and functions as various processing units. The PLD is a processor whose circuit configuration can be changed after manufacturing, such as a field programmable gate array (FPGA). The dedicated electric circuit is a processor that has a dedicated circuit configuration designed to perform a specific process, such as an application specific integrated circuit (ASIC).

One processing unit may be configured of one of these various processors, or may be configured of a combination of two or more processors of the same type or different types (for example, a combination of a plurality of FPGAs or a combination of a CPU and an FPGA). In addition, a plurality of processing units may be configured of one processor. As an example in which the plurality of processing units are configured of one processor, first, one processor is configured of a combination of one or more CPUs and software and this processor functions as the plurality of processing units. Second, as typified by a system on chip (SoC) or the like, a processor that realizes the functions of the entire system including the plurality of processing units by using one IC chip is used. As described above, the various processing units are configured using one or more of the various processors as a hardware structure.

More specifically, the hardware structure of these various processors is an electric circuit (circuitry) in which circuit elements such as semiconductor elements are combined.

The present invention is not limited to the above-described embodiment, and it is needless to say that various configurations can be adopted without departing from the scope of the present invention. In addition to the program, the present invention extends to a computer-readable storage medium that stores the program in a non-temporary manner.

**Claims**

1. An information processing apparatus(20) comprising:

   a processor (23),
   wherein the processor (23) is configured to

   acquire a plurality of unknown waveform data (UD) of which a determination result of superiority or inferiority based on similarity to an ideal waveform (GC) is unknown, the unknown waveform data being output from a plurality of electrodes (41) included in a microelectrode array (40),
   **characterised in that** the processor is further configured to perform a determination of the superiority or inferiority for each of the plurality of unknown waveform data (UD) based on a plurality of teacher waveform data (TD) to which the determination result of the superiority or inferiority is linked,
   perform a process of selecting an electrode (41) from which a waveform with the highest similarity to the ideal waveform is output from the plurality of electrodes (41) included in the microelectrode array (40), as a target electrode, and
   output the superiority or inferiority of the plurality of unknown waveform data (UD) in a comparable manner.

2. The information processing apparatus (20) according to claim 1,
   wherein the processor (23) is configured to

   perform clustering on a set including the plurality of teacher waveform data (TD) and the plurality of unknown waveform data (UD), and
   perform the determination of the superiority or inferiority by obtaining, for each of clusters (CL) including at least one of the plurality of unknown waveform data (UD), a probability (SC) that the unknown waveform data (UD) has a superior determination, as a result of the clustering.

3. The information processing apparatus (20) according to claim 2,
   wherein the processor (23) is configured to obtain the probability (SC) based on the number of the teacher waveform data (TD) with a superior determination and the number of the teacher waveform data (TD) with a superior determination and an inferior determination, for each of the clusters (CL).

4. The information processing apparatus (20) according to claim 3,

   wherein the probability (SC) is represented by a value obtained by dividing the number of the teacher waveform data (TD) with the superior determination by the number of the teacher waveform data (TD) with the superior determination and the inferior determination, and
   the processor (23) is configured to rank and output the superiority or inferiority of the plurality of unknown waveform data (UD) based on the probability (SC) in a comparable manner.

5. The information processing apparatus (20) according to any one of claims 2 to 4,
   wherein the processor (23) is configured to perform the clustering by a k-medoids method or a k-means method.

6. The information processing apparatus (20) according to claim 5,
   wherein the processor (23) is configured to perform a filtering process of excluding unknown waveform data (UD) that does not satisfy an evaluation criterion from the set or lowering a rank in terms of the superiority or inferiority.

7. The information processing apparatus (20) according to claim 1,
   wherein the processor (23) is configured to input the unknown waveform data (UD) to a neural network (70) that has been trained through machine learning based on the teacher waveform data (TD), and is configured to perform the determination of the superiority or inferiority based on a result output from the neural network (70).

8. The information processing apparatus (20) according to claim 1,
   wherein the processor (23) is configured to input the unknown waveform data (UD) to an encoder of an auto-encoder (80) that has been trained through machine learning based on the teacher waveform data (TD) with a superior determination, and then is configured to perform the determination of the superiority or inferiority based on a difference between waveform data restored by a decoder and the unknown waveform data (UD) input to the encoder.

9. The information processing apparatus (20) according to any one of claims 1 to 8,
   configured to receivethe unknown waveform data (UD) whichis a pulse signal output by a cell.

10. The information processing apparatus (20) according to claim 9,
wherein the cell is a myocardial cell.

11. A computer-implemented information processing method comprising:

Acquiring a plurality of unknown waveform data (UD) of which a determination result of superiority or inferiority based on similarity to an ideal waveform (GC) is unknown, the unknown waveform data being output from a plurality of electrodes (41) included in a microelectrode array (40);
**characterised by** performing a determination of the superiority or inferiority (S13) for each of the plurality of unknown waveform data (UD) through machine learning based on a plurality of teacher waveform data (TD) to which the determination result of the superiority or inferiority is linked;
perform a process of selecting an electrode (41) from which a waveform with the highest similarity to the ideal waveform is output from the plurality of electrodes (41) included in the microelectrode array (40), as a target electrode; and
outputting the superiority or inferiority of the plurality of unknown waveform data (UD) in a comparable manner.

12. A computer-readable storage medium storing a program causing a computer to execute:

an acquisition process of acquiring a plurality of unknown waveform data (UD) of which a determination result of superiority or inferiority (S13) based on similarity to an ideal waveform (GC) is unknown, the unknown waveform data being output from a plurality of electrodes (41) included in a microelectrode array (40);
**characterised by** a determination process of performing a determination of the superiority or inferiority (S13) for each of the plurality of unknown waveform data (UD) through machine learning based on a plurality of teacher waveform data (TD) to which the determination result of the superiority or inferiority is linked;
perform a process of selecting an electrode (41) from which a waveform with the highest similarity to the ideal waveform is output from the plurality of electrodes (41) included in the microelectrode array (40), as a target electrode, and
an output process of outputting the superiority or inferiority of the plurality of unknown waveform data (UD) in a comparable manner.

**Patentansprüche**

1. Informationsverarbeitungsvorrichtung (20), umfassend:

einen Prozessor (23),
wobei der Prozessor (23) so konfiguriert ist, dass er
mehrere unbekannte Wellenformdaten (UD), deren Bestimmungsergebnis von Überlegenheit oder Unterlegenheit auf der Grundlage von Ähnlichkeit zu einer idealen Wellenform (GC) unbekannt ist, erfasst, wobei die unbekannten Wellenformdaten von mehreren Elektroden (41), die in einer Mikroelektrodenanordnung (40) enthalten sind, ausgegeben werden,
**dadurch gekennzeichnet, dass** der Prozessor ferner so konfiguriert ist, dass er:

eine Bestimmung der Überlegenheit oder Unterlegenheit für jede der mehreren unbekannten Wellenformdaten (UD) auf der Grundlage von mehreren Lehrer-Wellenformdaten (TD), denen das Bestimmungsergebnis der Überlegenheit oder Unterlegenheit zugeordnet ist, durchführt,
einen Prozess des Auswählens einer Elektrode (41), von der eine Wellenform mit der höchsten Ähnlichkeit zu der idealen Wellenform ausgegeben wird, aus den mehreren Elektroden (41), die in der Mikroelektrodenanordnung (40) enthalten sind, als eine Ziel-Elektrode durchführt, und
die Überlegenheit oder Unterlegenheit der mehreren unbekannten Wellenformdaten (UD) auf eine vergleichbare Weise ausgibt.

2. Informationsverarbeitungsvorrichtung (20) nach Anspruch 1,

wobei der Prozessor (23) so konfiguriert ist, dass er
Clustern an einem Satz, der die mehreren Lehrer-Wellenformdaten (TD) und die mehreren unbekannten Wellenformdaten (UD) enthält, durchführt, und
die Bestimmung der Überlegenheit oder Unterlegenheit dadurch durchführt, dass er als ein Ergebnis des Clusterns für jeden von Clustern (CL), die mindestens eine der mehreren unbekannten Wellenformdaten (UD) enthalten, eine Wahrscheinlichkeit (SC) erhält, dass die unbekannten Wellenformdaten (UD) eine Überlegenheitsbestimmung aufweisen.

3. Informationsverarbeitungsvorrichtung (20) nach Anspruch 2,
wobei der Prozessor (23) so konfiguriert ist, dass er für jeden der Cluster (CL) die Wahrscheinlichkeit (SC) auf der Grundlage der Anzahl der Lehrer-Wellenformdaten (TD) mit einer Überlegenheitsbestimmung und der Anzahl der Lehrer-Wellenformdaten (TD) mit einer Überlegenheitsbestimmung und einer Unterlegenheitsbestimmung erhält.

4. Informationsverarbeitungsvorrichtung (20) nach Anspruch 3,
wobei die Wahrscheinlichkeit (SC) durch einen Wert dargestellt wird, der durch Dividieren der Anzahl der Lehrer-Wellenformdaten (TD) mit der Überlegenheitsbestimmung durch die Anzahl der Lehrer-Wellenformdaten (TD) mit der Überlegenheitsbestimmung und der Unterlegenheitsbestimmung erhalten wird, und der Prozessor (23) so konfiguriert ist, dass er die Überlegenheit oder Unterlegenheit der mehreren unbekannten Wellenformdaten (UD) auf der Grundlage der Wahrscheinlichkeit (SC) auf eine vergleichbare Weise bewertet und ausgibt.

5. Informationsverarbeitungsvorrichtung (20) nach einem der Ansprüche 2 bis 4,
wobei der Prozessor (23) so konfiguriert ist, dass er das Clustern durch ein k-Medoids-Verfahren oder ein k-Means-Verfahren durchführt.

6. Informationsverarbeitungsvorrichtung (20) nach Anspruch 5,
wobei der Prozessor (23) so konfiguriert ist, dass er einen Filterprozess des Ausschließens von unbekannten Wellenformdaten (UD), die ein Bewertungskriterium nicht erfüllen, aus dem Satz oder des Absenkens eines Rangs in Bezug auf die Überlegenheit oder Unterlegenheit durchführt.

7. Informationsverarbeitungsvorrichtung (20) nach Anspruch 1,
wobei der Prozessor (23) so konfiguriert ist, dass er die unbekannten Wellenformdaten (UD) in ein neuronales Netzwerk (70) eingibt, das auf der Grundlage der Lehrer-Wellenformdaten (TD) durch maschinelles Lernen trainiert worden ist, und so konfiguriert ist, dass er die Bestimmung der Überlegenheit oder Unterlegenheit auf der Grundlage eines von dem neuronalen Netzwerk (70) ausgegebenen Ergebnisses durchführt.

8. Informationsverarbeitungsvorrichtung (20) nach Anspruch 1,
wobei der Prozessor (23) so konfiguriert ist, dass er die unbekannten Wellenformdaten (UD) in einen Kodierer eines Auto-Kodierers (80) eingibt, der auf der Grundlage der Lehrer-Wellenformdaten (TD) mit einer Überlegenheitsbestimmung durch maschinelles Lernen trainiert worden ist, und dann so konfiguriert ist, dass er die Bestimmung der Überlegenheit oder Unterlegenheit auf der Grundlage eines Unterschieds zwischen Wellenformdaten, die durch einen Dekodierer wiederhergestellt worden sind, und den unbekannten Wellenformdaten (UD), die in den Kodierer eingegeben worden sind, durchführt.

9. Informationsverarbeitungsvorrichtung (20) nach einem der Ansprüche 1 bis 8,
die so konfiguriert ist, dass sie die unbekannten Wellenformdaten (UD) empfängt, die ein von einer Zelle ausgegebenes Impulssignal sind.

10. Informationsverarbeitungsvorrichtung (20) nach Anspruch 9,
wobei die Zelle eine Herzmuskelzelle ist.

11. Computerimplementiertes Informationsverarbeitungsverfahren, umfassend:

Erfassen von mehreren unbekannten Wellenformdaten (UD), deren Bestimmungsergebnis von Überlegenheit oder Unterlegenheit auf der Grundlage von Ähnlichkeit zu einer idealen Wellenform (GC) unbekannt ist, wobei die unbekannten Wellenformdaten von mehreren Elektroden (41), die in einer Mikroelektrodenanordnung (40) enthalten sind, ausgegeben werden;
**dadurch gekennzeichnet, dass** es durchführt:

eine Bestimmung der Überlegenheit oder Unterlegenheit (S13) für jede der mehreren unbekannten Wellenformdaten (UD) durch maschinelles Lernen auf der Grundlage von mehreren Lehrer-Wellenformdaten (TD), denen das Bestimmungsergebnis der Überlegenheit oder Unterlegenheit zugeordnet ist;
einen Prozess des Auswählens einer Elektrode (41), von der eine Wellenform mit der höchsten Ähnlichkeit zu der idealen Wellenform ausgegeben wird, aus den mehreren Elektroden (41), die in der Mikroelektrodenanordnung (40) enthalten sind, als eine Ziel-Elektrode durchführt; und
die Überlegenheit oder Unterlegenheit der mehreren unbekannten Wellenformdaten (UD) auf eine vergleichbare Weise ausgibt.

12. Computerlesbares Speichermedium, das ein Programm speichert, das einen Computer veranlasst, auszuführen:

einen Erfassungsprozess des Erfassens von mehreren unbekannten Wellenformdaten (UD), von denen ein Bestimmungsergebnis von Überlegenheit oder Unterlegenheit (S13) auf der Grundlage von Ähnlichkeit zu einer idealen Wellenform (GC) unbekannt ist, wobei die unbekannten Wellenformdaten von mehreren Elektroden (41), die in einer Mikroelektrodenanordnung (40) enthalten sind, ausgegeben werden;
**gekennzeichnet durch**:

einen Bestimmungsprozess des Durchführens einer Bestimmung der Überlegenheit oder Unterlegenheit (S13) für jede der mehreren unbekannten Wellenformdaten (UD) durch maschinelles Lernen auf der Grundlage von mehreren Lehrer-Wellenformdaten (TD), denen das Bestimmungsergebnis der Überlegenheit oder Unterlegenheit zugeordnet ist;
Durchführen eines Prozesses des Auswählens einer Elektrode (41), von der eine Wellenform mit der höchsten Ähnlichkeit zu der idealen Wellenform ausgegeben wird, aus den mehreren Elektroden (41), die in der Mikroelektrodenanordnung (40) enthalten sind, als eine Ziel-Elektrode; und
einen Ausgabeprozess des Ausgebens der Überlegenheit oder Unterlegenheit der mehreren unbekannten Wellenformdaten (UD) auf eine vergleichbare Weise.

## Revendications

1. Appareil de traitement d'informations (20) comprenant :

un processeur (23),
dans lequel le processeur (23) est configuré pour
acquérir une pluralité de données de forme d'onde inconnues (UD) dont un résultat de détermination de supériorité ou d'infériorité sur la base d'une similarité à une forme d'onde idéale (GC) est inconnu, les données de forme d'onde étant émises par une pluralité d'électrodes (41) incluses dans un réseau de microélectrodes (40),
**caractérisé en ce que** le processeur est en outre configuré pour :

effectuer une détermination de la supériorité ou de l'infériorité pour chacune de la pluralité de données de forme d'onde inconnues (UD) sur la base d'une pluralité de données de forme d'onde d'enseignant (TD) auxquelles le résultat de détermination de la supériorité ou de l'infériorité est lié,
effectuer un processus de sélection d'une électrode (41) à partir de laquelle une forme d'onde avec la plus grande similarité à la forme d'onde idéale est émise parmi la pluralité d'électrodes (41) incluses dans le réseau de microélectrodes (40), en tant qu'électrode cible, et
émettre la supériorité ou l'infériorité de la pluralité de données de forme d'onde inconnues (UD) de manière comparable.

2. Appareil de traitement d'informations (20) selon la revendication 1,

dans lequel le processeur (23) est configuré pour
effectuer un regroupement sur un ensemble incluant la pluralité de données de forme d'onde d'enseignant (TD) et la pluralité de données de forme d'onde inconnues (UD), et
effectuer la détermination de la supériorité ou de l'infériorité en obtenant, pour chacun des clusters (CL) incluant au moins une de la pluralité de données de forme d'onde inconnues (UD), une probabilité (SC) que les données de forme d'onde inconnues (UD) aient une détermination supérieure, à la suite du regroupement.

3. Appareil de traitement d'informations (20) selon la revendication 2,
dans lequel le processeur (23) est configuré pour obtenir la probabilité (SC) sur la base du nombre de données de forme d'onde d'enseignant (TD) avec une détermination supérieure et du nombre de données de forme d'onde d'apprentissage (TD) avec une détermination supérieure et une détermination inférieure, pour chacun des clusters (CL).

4. Appareil de traitement d'informations (20) selon la revendication 3,

dans lequel la probabilité (SC) est représentée par une valeur obtenue en divisant le nombre de données de forme d'onde d'apprentissage (TD) avec la détermination supérieure par le nombre de données de forme d'onde d'apprentissage (TD) avec la détermination supérieure et la détermination inférieure, et
le processeur (23) est configuré pour classer et émettre la supériorité ou l'infériorité de la pluralité de données de forme d'onde inconnues (UD) sur la base de la probabilité (SC) de manière comparable.

5. Appareil de traitement d'informations (20) selon l'une quelconque des revendications 2 à 4,
dans lequel le processeur (23) est configuré pour effectuer le regroupement par une méthode de k-medoids ou une méthode de k-moyennes.

6. Appareil de traitement d'informations (20) selon la revendication 5,
dans lequel le processeur (23) est configuré pour effectuer un processus de filtrage consistant à exclure des données de forme d'onde inconnues (UD) qui ne satisfont pas à un critère d'évaluation de l'ensemble ou à abaisser un rang en termes de supériorité ou d'infériorité.

7. Appareil de traitement d'informations (20) selon la revendication 1,
dans lequel le processeur (23) est configuré pour entrer les données de forme d'onde inconnues (UD) dans un réseau neuronal (70) qui a été entraîné par apprentissage automatique sur la base des données de forme d'onde d'enseignant (TD), et est configuré pour effectuer la détermination de la supériorité ou de l'infériorité sur la base d'un résultat émis du réseau neuronal (70).

8. Appareil de traitement d'informations (20) selon la revendication 1,
dans lequel le processeur (23) est configuré pour entrer les données de forme d'onde inconnues (UD) dans un codeur d'un autoencodeur (80) qui a été entraîné par apprentissage automatique sur la base des données de forme d'onde d'enseignant (TD) avec une détermination supérieure, puis est configuré pour effectuer la détermination de la supériorité ou de l'infériorité sur la base d'une différence entre des données de forme d'onde restaurées par un décodeur et les données de forme d'onde inconnues (UD) entrées dans le codeur.

9. Appareil de traitement d'informations (20) selon l'une quelconque des revendications 1 à 8,
configuré pour recevoir les données de forme d'onde inconnues (UD) qui sont un signal d'impulsion émis par une cellule.

10. Appareil de traitement d'informations (20) selon la revendication 9,
dans lequel la cellule est une cellule myocardique.

11. Procédé de traitement d'informations mis en œuvre par ordinateur comprenant :

acquérir une pluralité de données de forme d'onde inconnues (UD) dont un résultat de détermination de supériorité ou d'infériorité sur la base d'une similarité à une forme d'onde idéale (GC) est inconnu, les données de forme d'onde inconnues étant émises par une pluralité d'électrodes (41) incluses dans un réseau de microélectrodes (40) ;
**caractérisé par** l'exécution de une détermination de la supériorité ou de l'infériorité (S13) pour chacune de la pluralité de données de forme d'onde inconnues (UD) par apprentissage automatique sur la base d'une pluralité de données de forme d'onde d'enseignant (TD) auxquelles le résultat de détermination de la supériorité ou de l'infériorité est lié ;
l'exécution d'un processus de sélection d'une électrode (41) à partir de laquelle une forme d'onde avec la plus grande similarité à la forme d'onde idéale est émise parmi la pluralité d'électrodes (41) incluses dans le réseau de microélectrodes (40), en tant qu'électrode cible ; et
la production de la supériorité ou l'infériorité de la pluralité de données de forme d'onde inconnues (UD) de manière comparable.

12. Support de stockage lisible par ordinateur stockant un programme amenant un ordinateur à exécuter :

un processus d'acquisition consistant à acquérir une pluralité de données de forme d'onde inconnues (UD) dont un résultat de détermination de supériorité ou d'infériorité (S13) sur la base d'une similarité à une forme d'onde idéale (GC) est inconnu, les données de forme d'onde inconnues étant émises par une pluralité d'électrodes (41) incluses dans un réseau de microélectrodes (40) ;
**caractérisé par**
un processus de détermination consistant à effectuer une détermination de la supériorité ou de l'infériorité (S13) pour chacune de la pluralité de données de forme d'onde inconnues (UD) par apprentissage automatique sur la base d'une pluralité de données de forme d'onde d'enseignant (TD) auxquelles le résultat de détermination de la supériorité ou de l'infériorité est lié ;
l'exécution d'un processus de sélection d'une électrode (41) à partir de laquelle une forme d'onde avec la plus grande similarité à la forme d'onde idéale est émise parmi la pluralité d'électrodes (41) incluses dans le réseau de microélectrodes (40), en tant qu'électrode cible ; et
un processus de sortie consistant à émettre la supériorité ou l'infériorité de la pluralité de données de forme d'onde inconnues (UD) de manière comparable.

FIG. 1

EP 4 296 347 B1

# FIG. 2

FIG. 3

FIG. 4

# FIG. 5

# FIG. 6

CULTURE APPARATUS ~10

UNKNOWN WAVEFORM DATA ~UD

~20

PROCESSOR ~23

DATA ACQUISITION UNIT ~60

INPUT UNIT ~22

DETERMINATION UNIT ~61

OUTPUT UNIT ~62

MEMORY ~24

PROGRAM ~28

TEACHER WAVEFORM DATA ~TD

DISPLAY UNIT ~21

# FIG. 7

# FIG. 8

UNKNOWN WAVEFORM DATA FOR ONE WELL

CH1 CH2 CH3 CH4

CH5 CH6 CH7 CH8

CH9 CH10 CH11 CH12

CH13 CH14 CH15 CH16

# FIG. 9

DETERMINATION PROCESS START

S10
CREATE SET INCLUDING TEACHER WAVEFORM DATA
AND UNKNOWN WAVEFORM DATA

S11
PERFORM CLUSTERING BY k-medoids METHOD

S12
SPECIFY CLUSTER INCLUDING UNKNOWN WAVEFORM DATA

S13
DETERMINE SUPERIORITY
OR INFERIORITY FOR EACH UNKNOWN WAVEFORM DATA

S14
RANK UNKNOWN WAVEFORM DATA BASED ON
DETERMINATION RESULT OF SUPERIORITY OR INFERIORITY

S15
PLURALITY OF FIRST-RANKED
UNKNOWN WAVEFORM DATA EXIST?　　NO

YES

S16
RE-CLUSTER CLUSTER
INCLUDING FIRST-RANKED UNKNOWN WAVEFORM DATA

END

# FIG. 10

SET

~TD

| TEACHER WAVEFORM DATA | | | | | | |
|---|---|---|---|---|---|---|
| DATA NAME | TIME | | | | | SUPERIORITY OR INFERIORITY |
| | t1 | t2 | t3 | ・・・ | tn | |
| T1 | 0.0001 | 0.0002 | 0.0004 | ・・・ | 0.0001 | 0 |
| T2 | ・・・ | ・・・ | ・・・ | ・・・ | ・・・ | 0 |
| T3 | ・・・ | ・・・ | ・・・ | ・・・ | ・・・ | 0 |
| T4 | ・・・ | ・・・ | ・・・ | ・・・ | ・・・ | 1 |
| T5 | ・・・ | ・・・ | ・・・ | ・・・ | ・・・ | 0 |
| ⋮ | ⋮ | ⋮ | ⋮ | ⋮ | ⋮ | ⋮ |
| T40 | ・・・ | ・・・ | ・・・ | ・・・ | ・・・ | 1 |
| ⋮ | ⋮ | ⋮ | ⋮ | ⋮ | ⋮ | ⋮ |
| Tm | ・・・ | ・・・ | ・・・ | ・・・ | ・・・ | 0 |

GC (T4)

GC (T40)

~UD

| UNKNOWN WAVEFORM DATA | | | | | |
|---|---|---|---|---|---|
| DATA NAME | TIME | | | | |
| | t1 | t2 | t3 | ・・・ | tn |
| U1 | 0.0001 | 0.0003 | 0.0004 | ・・・ | 0.0002 |
| U2 | ・・・ | ・・・ | ・・・ | ・・・ | ・・・ |
| U3 | ・・・ | ・・・ | ・・・ | ・・・ | ・・・ |
| U4 | ・・・ | ・・・ | ・・・ | ・・・ | ・・・ |
| U5 | ・・・ | ・・・ | ・・・ | ・・・ | ・・・ |
| ⋮ | ⋮ | ⋮ | ⋮ | ⋮ | ⋮ |
| U16 | ・・・ | ・・・ | ・・・ | ・・・ | ・・・ |

FIG. 11

TEACHER WAVEFORM DATA

| DATA NAME | TIME | | | | | SUPERIORITY OR INFERIORITY |
|---|---|---|---|---|---|---|
| | t1 | t2 | t3 | $\cdots$ | tn | |
| T1 | 0.0001 | 0.0002 | 0.0004 | $\cdots$ | 0.0001 | 0 |
| T2 | $\cdots$ | $\cdots$ | $\cdots$ | $\cdots$ | $\cdots$ | 0 |
| T3 | $\cdots$ | $\cdots$ | $\cdots$ | $\cdots$ | $\cdots$ | 0 |
| T4 | $\cdots$ | $\cdots$ | $\cdots$ | $\cdots$ | $\cdots$ | 1 |
| T5 | $\cdots$ | $\cdots$ | $\cdots$ | $\cdots$ | $\cdots$ | 0 |
| $\vdots$ | $\vdots$ | $\vdots$ | $\vdots$ | $\vdots$ | $\vdots$ | $\vdots$ |
| T40 | $\cdots$ | $\cdots$ | $\cdots$ | $\cdots$ | $\cdots$ | 1 |
| $\vdots$ | $\vdots$ | $\vdots$ | $\vdots$ | $\vdots$ | $\vdots$ | $\vdots$ |
| T96 | $\cdots$ | $\cdots$ | $\cdots$ | $\cdots$ | $\cdots$ | 0 |
| U1 | 0.0001 | 0.0003 | 0.0004 | $\cdots$ | 0.0002 | |
| U2 | $\cdots$ | $\cdots$ | $\cdots$ | $\cdots$ | $\cdots$ | |
| U3 | $\cdots$ | $\cdots$ | $\cdots$ | $\cdots$ | $\cdots$ | |
| U4 | $\cdots$ | $\cdots$ | $\cdots$ | $\cdots$ | $\cdots$ | |
| U5 | $\cdots$ | $\cdots$ | $\cdots$ | $\cdots$ | $\cdots$ | |
| $\vdots$ | $\vdots$ | $\vdots$ | $\vdots$ | $\vdots$ | $\vdots$ | |
| U16 | $\cdots$ | $\cdots$ | $\cdots$ | $\cdots$ | $\cdots$ | |

CLUSTERING (k = 3) →

k CLUSTERS

CL1

| DATA NAME | SUPERIORITY OR INFERIORITY |
|---|---|
| T1 | 0 |
| T4 | 1 |
| T8 | 0 |
| T16 | 0 |
| $\vdots$ | $\vdots$ |
| T40 | 1 |
| $\vdots$ | $\vdots$ |
| T62 | 1 |
| $\vdots$ | $\vdots$ |
| T83 | 1 |
| $\vdots$ | $\vdots$ |
| U1 | |
| U4 | |
| U5 | |
| U10 | |
| U12 | |
| U16 | |

CL2

| DATA NAME | SUPERIORITY OR INFERIORITY |
|---|---|
| T2 | 0 |
| T5 | 0 |
| T7 | 0 |
| T9 | 0 |
| T10 | 0 |
| T12 | 0 |
| T13 | 0 |
| $\vdots$ | $\vdots$ |
| T34 | 1 |
| $\vdots$ | $\vdots$ |
| U2 | |
| U3 | |
| U7 | |
| U11 | |
| U13 | |
| U14 | |
| U15 | |

CL3

| DATA NAME | SUPERIORITY OR INFERIORITY |
|---|---|
| T3 | 0 |
| T6 | 0 |
| T7 | 0 |
| T11 | 0 |
| T14 | 0 |
| T15 | 0 |
| $\vdots$ | $\vdots$ |
| T28 | 1 |
| $\vdots$ | $\vdots$ |
| T59 | 1 |
| $\vdots$ | $\vdots$ |
| T75 | 1 |
| $\vdots$ | $\vdots$ |
| T93 | 1 |
| $\vdots$ | $\vdots$ |
| U6 | |
| U8 | |
| U9 | |

EP 4 296 347 B1

# FIG. 12

CLUSTERING
BY k-medoids METHOD

## FIG. 13

NT: THE NUMBER OF TEACHER WAVEFORM DATA
N1: THE NUMBER OF DATA WITH "1"

**CL1**

| DATA NAME | SUPERIORITY OR INFERIORITY |
|---|---|
| T1 | 0 |
| T4 | 1 |
| T8 | 0 |
| T16 | 0 |
| ⋮ | ⋮ |
| T40 | 1 |
| ⋮ | ⋮ |
| T62 | 1 |
| ⋮ | ⋮ |
| T83 | 1 |
| ⋮ | ⋮ |
| U1 | |
| U4 | |
| U5 | |
| U10 | |
| U12 | |
| U16 | |

NT = 32 (T1 through T83 group), N1 = 4

$$SC = N1/NT = 0.125$$

**CL2**

| DATA NAME | SUPERIORITY OR INFERIORITY |
|---|---|
| T2 | 0 |
| T5 | 0 |
| T7 | 0 |
| T9 | 0 |
| T10 | 0 |
| T12 | 0 |
| T13 | 0 |
| ⋮ | ⋮ |
| T34 | 1 |
| ⋮ | ⋮ |
| U2 | |
| U3 | |
| U7 | |
| U11 | |
| U13 | |
| U14 | |
| U15 | |

NT = 48 (T2 through T34 group), N1 = 1

$$SC = N1/NT = 0.021$$

**CL3**

| DATA NAME | SUPERIORITY OR INFERIORITY |
|---|---|
| T3 | 0 |
| T6 | 0 |
| T7 | 0 |
| T11 | 0 |
| T14 | 0 |
| T15 | 0 |
| ⋮ | ⋮ |
| T28 | 1 |
| ⋮ | ⋮ |
| T59 | 1 |
| ⋮ | ⋮ |
| T75 | 1 |
| ⋮ | ⋮ |
| T93 | 1 |
| ⋮ | ⋮ |
| U6 | |
| U8 | |
| U9 | |

NT = 16 (T3 through T93 group), N1 = 4

$$SC = N1/NT = 0.250$$

EP 4 296 347 B1

# FIG. 14

| | DATA NAME | SCORE SC | RANKING |
|---|---|---|---|
| CL3 | U6 | 0.250 | 1 |
| | U8 | 0.250 | 1 |
| | U9 | 0.250 | 1 |
| CL1 | U1 | 0.125 | 4 |
| | U4 | 0.125 | 4 |
| | U5 | 0.125 | 4 |
| | U10 | 0.125 | 4 |
| | U12 | 0.125 | 4 |
| | U16 | 0.125 | 4 |
| CL2 | U2 | 0.021 | 10 |
| | U3 | 0.021 | 10 |
| | U7 | 0.021 | 10 |
| | U11 | 0.021 | 10 |
| | U13 | 0.021 | 10 |
| | U14 | 0.021 | 10 |
| | U15 | 0.021 | 10 |

THE NUMBER OF PIECES OF DATA RANKED FIRST IS THREE ⇨ RE-CLUSTER CL3 ($k = 3$)

# FIG. 15

| DATA NAME | SCORE SC | RANKING |
|---|---|---|
| U9 | 0.333 | 1 — GC |
| U8 | 0.125 | 2 |
| U6 | 0 | 3 |

# FIG. 16

~21

SELECTION RESULT OF GOLDEN CHANNEL

CH1　　CH2　　CH3　　CH4

CH5　　CH6　　CH7　　CH8

CH9　GC　CH10　CH11　CH12

CH13　CH14　CH15　CH16

# FIG. 17

EP 4 296 347 B1

## SET ~TD

| TEACHER WAVEFORM DATA | | | | | | | | | | | | | | | |
|---|---|---|---|---|---|---|---|---|---|---|---|---|---|---|---|
| DATA NAME | TIME | | | | | | | | | | | | | | SUPERIORITY OR INFERIORITY |
| | t1 | t2 | t3 | t4 | t5 | t6 | t7 | t8 | t9 | t10 | ... | t7249 | t7250 | t7251 | |
| T1-1 | 4.75E-08 | 8.05E-08 | 1.06E-07 | 1.32E-07 | 1.50E-07 | 1.54E-07 | 1.50E-07 | 1.43E-07 | 1.43E-07 | 1.65E-07 | ... | 1.43E-07 | 1.43E-07 | 1.65E-07 | 0 |
| T1-2 | 3.29E-08 | 8.41E-08 | 1.35E-07 | 1.79E-07 | 2.19E-07 | 2.52E-07 | 2.78E-07 | 3.00E-07 | 3.18E-07 | 3.44E-07 | ... | 3.00E-07 | 3.18E-07 | 3.44E-07 | 0 |
| T1-3 | -3.65E-09 | -7.31E-09 | -3.65E-09 | 1.09E-08 | 3.29E-08 | 5.85E-08 | 8.04E-08 | 1.06E-07 | 1.35E-07 | 1.61E-07 | ... | 1.06E-07 | 1.35E-07 | 1.61E-07 | 1 |
| ... | ... | ... | ... | ... | ... | ... | ... | ... | ... | ... | ... | ... | ... | ... | ... |
| T1-383 | -4.39E-08 | -8.77E-08 | -1.35E-07 | -1.83E-07 | -2.30E-07 | -2.71E-07 | -3.00E-07 | -3.07E-07 | -3.00E-07 | -2.85E-07 | ... | -3.07E-07 | -3.00E-07 | -2.85E-07 | 1 |
| T1-384 | -5.12E-08 | -8.78E-08 | -1.02E-07 | -9.87E-08 | -7.68E-08 | -4.75E-08 | -1.83E-08 | 3.66E-09 | 1.83E-08 | 2.93E-08 | ... | 3.66E-09 | 1.83E-08 | 2.93E-08 | 0 |
| T2-1 | 4.75E-08 | 8.05E-08 | 1.06E-07 | 1.32E-07 | 1.50E-07 | 1.54E-07 | 1.50E-07 | 1.43E-07 | 1.43E-07 | 1.65E-07 | ... | 1.43E-07 | 1.43E-07 | 1.65E-07 | 1 |
| T2-2 | 3.29E-08 | 8.41E-08 | 1.35E-07 | 1.79E-07 | 2.19E-07 | 2.52E-07 | 2.78E-07 | 3.00E-07 | 3.18E-07 | 3.44E-07 | ... | 3.00E-07 | 3.18E-07 | 3.44E-07 | 0 |
| T2-3 | -3.65E-09 | -7.31E-09 | -3.65E-09 | 1.09E-08 | 3.29E-08 | 5.85E-08 | 8.04E-08 | 1.06E-07 | 1.35E-07 | 1.61E-07 | ... | 1.06E-07 | 1.35E-07 | 1.61E-07 | 0 |
| ... | ... | ... | ... | ... | ... | ... | ... | ... | ... | ... | ... | ... | ... | ... | ... |
| T2-383 | -4.39E-08 | -8.77E-08 | -1.35E-07 | -1.83E-07 | -2.30E-07 | -2.71E-07 | -3.00E-07 | -3.07E-07 | -3.00E-07 | -2.85E-07 | ... | -3.07E-07 | -3.00E-07 | -2.85E-07 | 0 |
| T2-384 | -5.12E-08 | -8.78E-08 | -1.02E-07 | -9.87E-08 | -7.68E-08 | -4.75E-08 | -1.83E-08 | 3.66E-09 | 1.83E-08 | 2.93E-08 | ... | 3.66E-09 | 1.83E-08 | 2.93E-08 | 1 |
| T3-1 | 4.75E-08 | 8.05E-08 | 1.06E-07 | 1.32E-07 | 1.50E-07 | 1.54E-07 | 1.50E-07 | 1.43E-07 | 1.43E-07 | 1.65E-07 | | 1.43E-07 | 1.43E-07 | 1.65E-07 | 0 |
| T3-2 | 3.29E-08 | 8.41E-08 | 1.35E-07 | 1.79E-07 | 2.19E-07 | 2.52E-07 | 2.78E-07 | 3.00E-07 | 3.18E-07 | 3.44E-07 | | 3.00E-07 | 3.18E-07 | 3.44E-07 | 1 |
| T3-3 | -3.65E-09 | -7.31E-09 | -3.65E-09 | 1.09E-08 | 3.29E-08 | 5.85E-08 | 8.04E-08 | 1.06E-07 | 1.35E-07 | 1.61E-07 | ... | 1.06E-07 | 1.35E-07 | 1.61E-07 | 0 |
| ... | ... | ... | ... | ... | ... | ... | ... | ... | ... | ... | ... | ... | ... | ... | ... |
| T3-383 | -4.39E-08 | -8.77E-08 | -1.35E-07 | -1.83E-07 | -2.30E-07 | -2.71E-07 | -3.00E-07 | -3.07E-07 | -3.00E-07 | -2.85E-07 | ... | -3.07E-07 | -3.00E-07 | -2.85E-07 | 0 |
| T3-384 | -5.12E-08 | -8.78E-08 | -1.02E-07 | -9.87E-08 | -7.68E-08 | -4.75E-08 | -1.83E-08 | 3.66E-09 | 1.83E-08 | 2.93E-08 | ... | 3.66E-09 | 1.83E-08 | 2.93E-08 | 0 |

## ~UD

| UNKNOWN WAVEFORM DATA | | | | | | | | | | | | | | |
|---|---|---|---|---|---|---|---|---|---|---|---|---|---|---|
| DATA NAME | TIME | | | | | | | | | | | | | |
| | t1 | t2 | t3 | t4 | t5 | t6 | t7 | t8 | t9 | t10 | ... | t7249 | t7250 | t7251 |
| U1 | 4.75E-08 | 8.05E-08 | 1.06E-07 | 1.32E-07 | 1.50E-07 | 1.54E-07 | 1.50E-07 | 1.43E-07 | 1.43E-07 | 1.65E-07 | ... | 1.43E-07 | 1.43E-07 | 1.65E-07 |
| U2 | 3.29E-08 | 8.41E-08 | 1.35E-07 | 1.79E-07 | 2.19E-07 | 2.52E-07 | 2.78E-07 | 3.00E-07 | 3.18E-07 | 3.44E-07 | ... | 3.00E-07 | 3.18E-07 | 3.44E-07 |
| U3 | -3.65E-09 | -7.31E-09 | -3.65E-09 | 1.09E-08 | 3.29E-08 | 5.85E-08 | 8.04E-08 | 1.06E-07 | 1.35E-07 | 1.61E-07 | ... | 1.06E-07 | 1.35E-07 | 1.61E-07 |
| ... | ... | ... | ... | ... | ... | ... | ... | ... | ... | ... | ... | ... | ... | ... |
| U15 | -4.39E-08 | -8.77E-08 | -1.35E-07 | -1.83E-07 | -2.30E-07 | -2.71E-07 | -3.00E-07 | -3.07E-07 | -3.00E-07 | -2.85E-07 | | -3.07E-07 | -3.00E-07 | -2.85E-07 |
| U16 | -5.12E-08 | -8.78E-08 | -1.02E-07 | -9.87E-08 | -7.68E-08 | -4.75E-08 | -1.83E-08 | 3.66E-09 | 1.83E-08 | 2.93E-08 | ... | 3.66E-09 | 1.83E-08 | 2.93E-08 |

# FIG. 18

FIRST CLUSTERING (k = 10)

CL1

| DATA NAME | SUPERIORITY OR INFERIORITY |
|---|---|
| T1-1 | 0 |
| T1-2 | 0 |
| T2-383 | 0 |
| T2-384 | 1 |
| ... | ... |
| T3-125 | 0 |
| T3-381 | 1 |
| U2 | |

⇨ SC = 0.014

CL2

| DATA NAME | SUPERIORITY OR INFERIORITY |
|---|---|
| T1-8 | 0 |
| T1-15 | 0 |
| T2-245 | 0 |
| T2-251 | 1 |
| ... | ... |
| T3-265 | 0 |
| T3-380 | 1 |
| U5 | |

⇨ SC = 0.094

⋮

CL10

| DATA NAME | SUPERIORITY OR INFERIORITY |
|---|---|
| T1-32 | 0 |
| T1-45 | 0 |
| T2-298 | 0 |
| T2-300 | 1 |
| ... | ... |
| T3-378 | 0 |
| T3-384 | 1 |
| U15 | |
| U16 | |

⇨ SC = 0.070

| DATA NAME | SCORE SC | RANKING |
|---|---|---|
| U3 | 0.135 | 1 |
| U6 | 0.135 | 1 |
| U11 | 0.135 | 1 |
| U5 | 0.094 | 4 |
| U7 | 0.094 | 4 |
| U15 | 0.070 | 6 |
| U16 | 0.070 | 6 |
| U4 | 0.057 | 8 |
| U8 | 0.057 | 8 |
| U2 | 0.041 | 10 |
| U1 | 0.032 | 11 |
| U9 | 0.032 | 11 |
| U12 | 0.031 | 13 |
| U10 | 0.022 | 14 |
| U14 | 0.022 | 14 |
| U13 | 0.015 | 16 |

⎫ CL6

⇩

RE-CLUSTER CL6 (k = 3)

30

# FIG. 19

SECOND CLUSTERING (k = 3)

CL6

| DATA NAME | SUPERIORITY OR INFERIORITY |
|---|---|
| T2-34 | 1 |
| T2-35 | 0 |
| T2-85 | 1 |
| T2-97 | 1 |
| ... | ... |
| T3-221 | 0 |
| T3-234 | 0 |
| U6 | |
| U6 | |
| U11 | |

CL6-1

| DATA NAME | SUPERIORITY OR INFERIORITY |
|---|---|
| T2-35 | 0 |
| T2-85 | 0 |
| ... | ... |
| T3-221 | 1 |
| T3-234 | 0 |
| U3 | |

SC = 0.120

CL6-2

| DATA NAME | SUPERIORITY OR INFERIORITY |
|---|---|
| T2-42 | 0 |
| T2-48 | 0 |
| ... | ... |
| T3-213 | 1 |
| T3-215 | 0 |
| U6 | |

SC = 0.184

CL6-3

| DATA NAME | SUPERIORITY OR INFERIORITY |
|---|---|
| T2-34 | 0 |
| T2-97 | 0 |
| ... | ... |
| T3-217 | 1 |
| T3-220 | 0 |
| U11 | |

SC = 0.210

# FIG. 20

| DATA NAME | SCORE SC | RANKING |
|:---:|:---:|:---:|
| U11 | 0.210 | 1 |
| U6 | 0.184 | 2 |
| U3 | 0.120 | 3 |
| U5 | 0.094 | 4 |
| U7 | 0.094 | 4 |
| U15 | 0.070 | 6 |
| U16 | 0.070 | 6 |
| U4 | 0.057 | 8 |
| U8 | 0.057 | 8 |
| U2 | 0.041 | 10 |
| U1 | 0.032 | 11 |
| U9 | 0.032 | 11 |
| U12 | 0.031 | 13 |
| U10 | 0.022 | 14 |
| U14 | 0.022 | 14 |
| U13 | 0.015 | 16 |

GC

# FIG. 21

| | TEACHER WAVEFORM DATA | | | | | ~TD |
|---|---|---|---|---|---|---|
| DATA NAME | TIME | | | | | SUPERIORITY OR INFERIORITY |
| | t1 | t2 | t3 | ・・・ | tn | |
| T1 | 0.0001 | 0.0002 | 0.0004 | ・・・ | 0.0001 | 0 |
| T2 | ・・・ | ・・・ | ・・・ | ・・・ | ・・・ | 0 |
| T3 | ・・・ | ・・・ | ・・・ | ・・・ | ・・・ | 0 |
| T4 | ・・・ | ・・・ | ・・・ | ・・・ | ・・・ | 1 |
| T5 | ・・・ | ・・・ | ・・・ | ・・・ | ・・・ | 0 |
| ⋮ | | | | | | |

LABEL

~70

NEURAL NETWORK

INPUT LAYER

INTERMEDIATE LAYER

OUTPUT LAYER

t1 TO tn

OUTPUT VALUE

~71

ADJUSTMENT UNIT

EP 4 296 347 B1

# FIG. 22

# FIG. 23

# FIG. 24

AUTO-ENCODER ~80

TD

INPUT

OUTPUT

ONLY TEACHER WAVEFORM DATA
WITH SUPERIOR DETERMINATION

ENCODER    DECODER

## FIG. 25

EP 4 296 347 B1

# FIG. 26

AUTO-ENCODER ~80A

UD

INPUT

OUTPUT

ENCODER     DECODER

COMPARISON UNIT ~81

DIFFERENCE VALUE IS EQUAL TO
OR GREATER THAN CERTAIN VALUE

0

EP 4 296 347 B1

# FIG. 27

DETERMINATION PROCESS START

S10

CREATE SET INCLUDING TEACHER WAVEFORM DATA
AND UNKNOWN WAVEFORM DATA

S20

EXCLUDE UNKNOWN WAVEFORM DATA THAT
DOES NOT SATISFY EVALUATION CRITERIA FROM SET

S11

PERFORM CLUSTERING BY k-medoids METHOD

S12

SPECIFY CLUSTER INCLUDING UNKNOWN WAVEFORM DATA

S13

DETERMINE SUPERIORITY
OR INFERIORITY FOR EACH UNKNOWN WAVEFORM DATA

S14

RANK UNKNOWN WAVEFORM DATA BASED ON
DETERMINATION RESULT OF SUPERIORITY OR INFERIORITY

S15

PLURALITY OF FIRST-RANKED
UNKNOWN WAVEFORM DATA EXIST?

NO

YES

S16

RE-CLUSTER CLUSTER
INCLUDING FIRST-RANKED UNKNOWN WAVEFORM DATA

END

# FIG. 28

DETERMINATION PROCESS START

~S10

CREATE SET INCLUDING TEACHER WAVEFORM DATA
AND UNKNOWN WAVEFORM DATA

~S11

PERFORM CLUSTERING BY k-medoids METHOD

~S12

SPECIFY CLUSTER INCLUDING UNKNOWN WAVEFORM DATA

~S13

DETERMINE SUPERIORITY
OR INFERIORITY FOR EACH UNKNOWN WAVEFORM DATA

~S14

RANK UNKNOWN WAVEFORM DATA BASED ON
DETERMINATION RESULT OF SUPERIORITY OR INFERIORITY

~S30

LOWER RANK OF UNKNOWN WAVEFORM DATA
THAT DOES NOT SATISFY EVALUATION CRITERIA

~S15

PLURALITY OF FIRST-RANKED
UNKNOWN WAVEFORM DATA EXIST?     NO

YES     ~S16

RE-CLUSTER CLUSTER
INCLUDING FIRST-RANKED UNKNOWN WAVEFORM DATA

END

# FIG. 29

| | DATA NAME | SCORE SC | RANKING |
|---|---|---|---|
| CL3 | U6 | 0.250 | 1 |
| | U8 | 0.250 | 1 |
| | U9 | 0.250 | 1 |
| CL1 | U1 | 0.125 | 4 |
| | U4 | 0.125 | 4 |
| | U5 | 0.125 | 4 |
| | U10 | 0.125 | 4 |
| | U12 | 0.125 | 4 |
| | U16 | 0.125 | 4 |
| CL2 | U2 | 0.021 | 10 |
| | U3 | 0.021 | 10 |
| | U7 | 0.021 | 10 |
| | U11 | 0.021 | 10 |
| | U13 | 0.021 | 10 |
| | U14 | 0.021 | 10 |
| | U15 | 0.021 | 10 |

⇒

| DATA NAME | SCORE SC | RANKING | EVALUATION CRITERIA P1max ≥ 200 P1min ≤ −200 | | A2 ≥ 15 FPDcF ≥ 340 | | DETERMINATION 1: SUPERIORITY 0: INFERIORITY |
|---|---|---|---|---|---|---|---|
| | | | P1max ($\mu$V) | P1min ($\mu$V) | A2 ($\mu$V) | FPDcF (msec) | |
| U6 | 0.250 | 1 | 221 | * −197 | 16.3 | 360 | 0 |
| U8 | 0.250 | 1 | 233 | −211 | * 14.8 | 348 | 0 |
| U9 | 0.250 | 1 | 212 | −203 | 17.6 | 355 | 1 |
| U1 | 0.125 | 4 | 200 | −202 | 15.4 | 352 | 1 |
| U4 | 0.125 | 4 | 220 | −211 | * 14.9 | 365 | 0 |
| U5 | 0.125 | 4 | 210 | −205 | 15.3 | 358 | 1 |
| U10 | 0.125 | 4 | 235 | −202 | 15.2 | 364 | 1 |
| U12 | 0.125 | 4 | 215 | −210 | * 13.8 | 343 | 0 |
| U16 | 0.125 | 4 | 225 | −207 | * 14.1 | 356 | 0 |
| U2 | 0.021 | 10 | * 198 | * −187 | * 9.6 | 342 | 0 |
| U3 | 0.021 | 10 | * 189 | * −196 | * 8.8 | * 338 | 0 |
| U7 | 0.021 | 10 | * 190 | * −178 | * 7.8 | * 335 | 0 |
| U11 | 0.021 | 10 | 201 | * −175 | * 8.8 | 349 | 0 |
| U13 | 0.021 | 10 | * 185 | * −189 | * 9.1 | * 336 | 0 |
| U14 | 0.021 | 10 | * 195 | * −192 | * 7.5 | 342 | 0 |
| U15 | 0.021 | 10 | * 199 | * −186 | * 8.1 | * 338 | 0 |

GC

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019131806 A
- WO 2020226879 A1
- WO 2019196076 A1
- US 20180372724 A1


**Non-patent literature cited in the description**

- *Seeding and culturing of iCell (registered trademark) myocardial cells 2.0 on an MED plate*, 27 January 2021, <https://alphamedsci.com/download/protocols/dissociated5_190625%20(J).pdf>
- **CANTWELL et al.** *Rethinking multiscale cardiac electrophysiology with machine learning and predictive modelling*, 2019